(19) Europäisches Patentamt
European Patent Office
Office européen des brevets

(11) EP 1 205 178 A2

(12) **EUROPÄISCHE PATENTANMELDUNG**

(43) Veröffentlichungstag:
**15.05.2002 Patentblatt 2002/20**

(51) Int Cl.7: **A61K 7/42**

(21) Anmeldenummer: **01126791.1**

(22) Anmeldetag: **09.11.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **09.11.2000 DE 10055588**

(71) Anmelder: **MERCK PATENT GmbH**
**64293 Darmstadt (DE)**

(72) Erfinder:
- **Buchholz, Herwig**
  **60599 Frankurt/ Main (DE)**
- **Poetsch, Eike**
  **64367 Mühlwald (DE)**
- **Pflücker, Frank**
  **64297 Darmstadt (DE)**
- **Anselmann, Ralf**
  **67305 Ramsen (DE)**
- **Rosskopf, Ralf**
  **64839 Münster (DE)**
- **Kirschbaum, Michael**
  **64331 Weiterstadt (DE)**

(74) Vertreter: **Grünecker, Kinkeldey,**
**Stockmair & Schwanhäusser Anwaltssozietät**
**Maximilianstrasse 58**
**80538 München (DE)**

(54) **Konjugat, dessen Herstellung und Verwendung**

(57) Die Erfindung betrifft ein Konjugat, das zur Herstellung von dermatologischen und kosmetischen Zusammensetzungen verwendet werden kann. Die Erfindung betrifft ebenfalls Verfahren zur Herstellung eines solchen Konjugats sowie dessen Verwendung.

EP 1 205 178 A2

## Beschreibung

Die vorliegende Erfindung betrifft ein Konjugat, das zur Herstellung von dermatologischen und kosmetischen Zusammensetzungen verwendet werden kann. Die Erfindung betrifft ebenfalls Verfahren zur Herstellung eines solchen Konjugats sowie dessen Verwendung.

Dermatologische oder kosmetische Zusammensetzungen werden z.B. verwendet, um die Haut vor schädlichen äußeren Einflüssen, wie z.B. vor Sonnenstrahlung, zu schützen. Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie z.B. Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verhindern können. Die in den pharmazeutischen oder kosmetischen Zubereitungen enthaltenen UV-Filter bilden auf der Oberfläche der Haut einen Film bzw. eine Schicht aus. Bekannte UV-Filter bzw. Sonnenschutzmittel wirken in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfaßt UVB-Strahlen mit einer Wellenlänge zwischen 280 und 320 nm, die bei der Bildung eines Sonnenerythems eine entscheidende Rolle spielen, sowie UVA-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Auf die Haut aufgebrachte Lichtschutzformulierungen haben die Aufgabe, die hautschädigenden Strahlungsanteile zurückzuhalten. Als Lichtfilter kommen anorganische oder organische Materialien in Frage.

Lichtschutzformulierungen auf der Basis organischer Lichtfilter enthalten organische Lichtfilter, die in Wasser und/oder Öl löslich sind oder die weder in Wasser noch in Öl löslich sind.

Lichtschutzformulierungen mit unlöslichen partikulären organischen Lichtfiltem werden z.B. in der WO 97/3643 beschrieben. Die Gruppe der unlöslichen organischen Lichtfilter ist jedoch auf wenige Verbindungsklassen beschränkt.

Lichtschutzformulierungen mit organischen Lichtfiltern, die in Wasser und/oder in Öl löslich sind, werden in den Veröffentlichungen DE-A-197 46 654, DE-A-197 55 504, EP-A-709 080, EP-A-775 698, EP-A-893 119 und US-A-5 882 632 beschrieben.

Die DE-A-197 46 654 beschreibt beispielsweise die Verwendung von 4,4-Diarylbutadienderivaten als lösliche organische Lichtfilter in Lichtschutzformulierungen zum Schutz der Haut gegen UVA-Strahlung.

Die zuvor genannten Lichtschutzformulierungen müssen die organischen Lichtfilter in einer hohen Konzentration enthalten, um einen ausreichenden Lichtschutz zu gewährleisten. Der gravierendste Nachteil organischer löslicher Lichtfilter besteht jedoch darin, dass sie auf Grund ihrer Löslichkeit gegebenenfalls in die Haut eindringen und Hautschädigungen oder Allergien verursachen können.

Die JP-A-11-255 630 beschreibt eine Lichtschutzformulierung zum Schutz der Haut gegen UVA-Strahlung, die ein Dibenzoylmethanderivat enthält, das auf einem mit einem Silikonpolymer beschichteten anorganischen Träger aufgebracht ist. Die Herstellung dieser Lichtschutzformulierung ist jedoch auf Grund der Vielzahl an Arbeitsschritten umständlich und zeitaufwendig. Weiterhin sind Dibenzoylmethanderivate nicht photostabil.

Dermatologische und kosmetische Zusammensetzungen können weiterhin eine Vielzahl von Wirkstoffen enthalten, wie z.B. organische Substanzen mit antioxidativen und/oder radikalinhibierenden Eigenschaften sowie Repellentien.

Generell ist eine Heterogenisierung der in den dermatologischen und kosmetischen Zusammensetzungen enthaltenen Wirkstoffe erstrebenswert, da u.a. eine Penetration in die Haut und eine möglicherweise daraus resultierende Hautschädigung oder Allergie verhindert werden kann.

Aufgabe der vorliegenden Erfindung ist es, ein Konjugat auf der Basis organischer Wirkstoffe bereitzustellen, das nicht in die Haut eindringen bzw. penetrieren kann.

Diese Aufgabe wird erfindungsgemäß durch die Bereitstellung eines Konjugats gelöst, das ein anorganisches Pigment und einen Wirkstoff auf der Basis organischer Verbindungen umfasst, der kovalent über eine Spacer-Gruppe an das anorganische Pigment gebunden ist. Das erfindungsgemäße Konjugat ist dadurch gekennzeichnet, dass die Spacer-Gruppe ein Element aus den Gruppen 3A, 4A, 3B, 4B, 5B und 6B des Periodensystem der Elemente enthält.

Die Erfindung stellt ebenfalls eine dermatologische oder kosmetische Zusammensetzung bereit, umfassend mindestens ein Konjugat der zuvor genannten Art und mindestens einen kosmetisch, pharmazeutisch und/oder dermatologisch verträglichen Träger und/oder Hilfsstoff.

Im Rahmen der vorliegenden Erfindung wird unter dem Begriff "Konjugat" das Produkt verstanden, das durch molekulare, d.h. kovalente Verknüpfung des Wirkstoffes mit dem anorganischen Pigment erhalten wird. Der Begriff "Wirkstoff" umfasst z.B. lichtabsorbierende organische Verbindungen, Substanzen mit antioxidativen und/oder radikalinhibierenden Eigenschaften, Repellentien, Konservierungsmittel und Derivate dieser Wirkstoffe, die kovalent über eine Spacer-Gruppe an das anorganische Pigment gebunden werden können. Der Wirkstoff oder das Derivat davon umfasst bevorzugt eine nucleophile Gruppe. Es ist bevorzugt, dass der Wirkstoff als solcher, d.h. ohne an das anorganische Pigment gebunden zu sein, wasserlöslich und/oder öllöslich ist.

Das erfindungsgemäße Konjugat enthält ein anorganisches Pigment. Im Rahmen der vorliegenden Erfindung wird unter dem Begriff "Pigment" ein Farb- oder Füllstoff verstanden, der im Anwendungsmedium unlöslich ist. Das anorganische Pigment in dem erfindungsgemäßen Konjugat ist bevorzugt ein Metall- oder Halbmetalloxid.

Beispiele für anorganische Pigmente umfassen Oxide, Silikate, Phosphate, Carbonate, Sulfate und Nitride, wobei Oxide bevorzugt verwendet werden.

Bevorzugte anorganische Pigmente umfassen Magnesiumoxid, Aluminiumoxid, Siliciumdioxid, Zinkoxid, Ceroxid, Titandioxid, Zirconiumoxid, Manganoxid, Boroxid, rotes oder schwarzes Eisenoxid, Talk, Kaolin, natürliche und synthetische Glimmermaterialien, wie z.B. Muscovit, Phlogopit, Lepidolit, Biotit und Vermiculit, Magnesiumcarbonat, Calciumcarbonat, Aluminiumsilikat, Bariumsilikat, Calciumsilikat, Magnesiumsilikat, Strontiumsilikat, Bariumsulfat, Calciumsulfat, Calciumphosphat, Fluorapatit, Hydroxyapatit, Keramikpulver, Bornitrid, Eisentitanat, Zeolith und Gemische davon. Siliciumdioxid, Titandioxid, Glimmer, Talk und Gemische der zuvor genannten Pigmente (im folgenden als "Mischpigmente" bezeichnet), wie z.B. Siliciumdioxid/Titandioxid, werden besonders bevorzugt verwendet.

Handelsüblich erhältliche anorganische Mischpigmente, die erfindungsgemäß verwendet werden können, umfassen Gemische von Titandioxid/Glimmer (Mica), Titandioxid/Glimmer/ Zinnoxid, Titandioxid/Glimmer/Eisenoxide, Titandioxid/Glimmer/Siliciumdioxid, Titandioxid/Glimmer/Karmin, Glimmer/Eisenoxide/Aluminiumoxid, Glimmer/Eisenoxide, Titandioxid/Glimmer/Zinkoxid, TItandloxid/Gilmmer/Bariumsulfat, Glimmer/Siliciumdioxid und Titandioxid/Eisenoxide/Siliciumdioxid. Diese Mischpigmente werden unter den Bezeichnungen Timiron®, Soloron®, Colorona®, Dichrona®, Microna®, Micronaspher® und Ronaspher® vertrieben.

Pigmente, die Licht streuen, wie z.B. Ronaspher® LDP, sowie Perlglanzpigmente können ebenfalls verwendet werden.

Die Silikate können eine ketten-, band- oder blattförmige Struktur besitzen. Silikate mit einer blattförmigen Struktur, wie z.B. Glimmer oder Talk, werden bevorzugt verwendet.

Die Form, in der die Verbindung des Metalls oder Halbmetalls vorliegt, ist nicht auf bestimmte Formen beschränkt.

Die Verbindung des Metalls oder Halbmetalls liegt bevorzugt in Form von sphärischen Teilchen vor. Geeignete Materialien auf der Basis von Siliciumdioxid umfassen handelsüblich erhältliche Produkte, die unter der Bezeichnung Monospher®, wie z.B. Monospher® 10 (Siliciumdioxid mit einer Partikelgröße von 10 nm), Monospher® 25 (Siliciumdioxid mit einer Partikelgröße von 25 nm), Monospher® 100 (Siliciumdioxid mit einer Partikelgröße von 100 nm) oder Monospher® 500 (Siliciumdioxid mit einer Partikelgröße von 500 nm), oder Ronaspher® (Siliciumdioxid mit einer Partikelgröße von 50 nm bis 3 µm) angeboten werden.

Die Herstellung von monodispersen kugelförmigen Oxidpartikeln ist bekannt. Entsprechend dem Verfahren, das in der EP-A-216 278 beschrieben ist, können monodisperse kugelförmige Oxidpartikel durch hydrolytische Polykondensation von Alkoxiden erhalten werden.

Andere bevorzugte Formen, in denen die Verbindungen des Metalls oder Halbmetalls vorliegen können, umfassen Nadeln und Flocken.

Die Wirkstoffe auf der Basis organischer Verbindungen, die erfindungsgemäß kovalent an das anorganische Pigment gebunden werden, umfassen z.B. lichtabsorbierende organische Verbindungen, Substanzen mit antioxidativen und/oder radikalinhibierenden Eigenschaften, Repellentien sowie Konservierungsmittel. Die erfindungsgemäß verwendeten Wirkstoffe sind jedoch nicht auf diese Wirkstoffe beschränkt.

Die lichtabsorbierenden organischen Verbindungen werden aus Verbindungen ausgewählt, die UV-Licht absorbieren. Dabei werden Verbindungen eingesetzt, die UV-Licht im UVB-Bereich, d.h. im Bereich von 280 bis 320 nm, und/oder im UVA-Bereich, d.h. im Bereich von 320 bis 400 nm, absorbieren.

Die UVB-Filter besitzen vorzugsweise ein Absorptionsmaximum im Bereich von 300 bis 320 nm und können aus bekannten Substanzen, die in der Fachliteratur beschrieben werden, ausgewählt werden. Beispiele umfassen Derivate von Aminobenzoesäure, Zimtsäure, Salicylsäure, Benzylidenkampfer, Phenylbenzimidazol, Diphenylacrylat, Triazin, Triazol und vinylgruppenhaltigen Amiden.

Beispiele für Aminobenzoesäurederivate umfassen 4-Aminobenzoesäure, 4-Aminobenzoesäure-2,3-dihydroxypropylester, 4-[Bis(2-hydroxypropyl)amino]benzoesäureethylester, 4-(Dimethylamino)benzoesäure-2-ethylhexylester (z.B. Eusolex® 6007) und ethoxylierten 4-Aminobenzoesäureethylester (z.B. Uvinul® P25).

Beispiele für Zimtsäurederivate umfassen Zimtsäureester, wie p-Methoxyzimtsäure-2-ethylhexylester (z.B. Eusolex® 2292), 4-Methoxyzimtsäureisopentylester, z.B. als Gemisch der Isomere (z.B. Neo Heliopan® E 1000), und

4-Methoxyzimtsäure-2-methylhexylester, sowie das Diethanolaminsalz von 4-Methoxy-Zimtsäure und Zimtsäurederivate, die in der US-A-5601811 und in der WO 97/851 beschrieben werden.

Zu den Salicylsäurederivaten zählen beispielsweise 2-Ethylhexylsalicylat (z.B. Eusolex® OS), 4-Isopropylbenzylsalicylat (z.B. Megasol®) und 3,3,5-Trimethylcyclohexylsalicylat (z.B. Eusolex® HMS).

Beispiele für Benzylidenkampferderivate umfassen 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)aniliniummethylsulfat (z.B. Mexoryl® SK) und $\alpha$-(2-Oxoborn-3-yliden)toluol-4-sulfonsäure (z.B. Mexoryl® SL).

Als Beispiele für Phenylbenzimidazolderivate können 2-Phenylbenzimdazol-5-sulfonsäure und ihre Kalium-, Natrium- und Triethanolaminsalze genannt werden (z.B. Eusolex® 232).

Spezielle Beispiele für Diphenylacrylatderivate umfassen 2-Cyan-3,3'-diphenylacrylsäure-2-ethylhexylester und 2-Cyan-3,3'-diphenylacrylsäureethylester.

Beispiele für Triazolderivate umfassen Benzotriazole, wie z.B. 2-(2-Hydroxy-5-methylphenyl)benzotriazol, sowie die Triazole, die in der EP-A-893119 beschrieben werden.

Spezielle Beispiele für Triazine umfassen 2,4,6-Tri-{-4-[(2-ethylhexyl)oxycarbonyl]-phenylamino}-1,3,5-triazin sowie die Verbindungen, die in der EP-A-893119 beschrieben werden. Weitere Beispiele umfassen Trianilin-Triazinderivate, wie sie in der US-A-5 332 568, EP-A-570838, EP-A-517104, US-A-5 252 323, WO 93/17002 und der WO 97/03642 offenbart werden, Hydroxyphenyltriazinderivate, wie sie in der EP-A-775698 beschrieben werden, sowie Bis-Resorcinoldialkylaminotriazine, wie sie beispielsweise in der EP-A-780382 offenbart werden.

Bevorzugte Beispiele für vinylgruppenhaltigen Amidderivate umfassen jene, die in der EP-A-893119 beschrieben werden.

Als UVA-Filtersubstanzen kommen vorzugsweise Verbindungen in Betracht, die ein Absorptionsmaximum im Bereich von 330 bis 360 nm besitzen. Es können beliebige bekannte UVA-Filtersubstanzen, wie beispielsweise Derivate von Benzophenon, Dibenzoylmethan, Dirarylbutadien und Triazin, verwendet werden.

Spezielle Beispiele für Benzophenonderivate umfassen 2-Hydroxy-4-methoxybenzophenon (z.B. Eusolex® 4360), 2-Hydroxy-4-methoxybenzophenon-5-sulfonsäure und ihr Natriumsalz (z.B. Uvinul® MS-40), sowie 8-(2,2'-Dihydroxy-4-methoxybenzophenon).

Spezielle Beispiele für Benzoylmethanderivate und Dibenzoylmethanderivate umfassen 1-(4-tert-Butylphenyl)-3-(4-methoxyphenyl)propan-1,3-dion (z.B. Eusolex® 9020) und 4-Isopropyldibenzoylmethan (z.B. Eusolex® 8020).

Beispiele für Diarylbutadienderivate umfassen die 4,4-Diarylbutadiene, die in der DE-A-197 46 654 beschrieben werden, insbesondere 4,4-Diphenylbutadien.

Spezielle Beispiele für Triazine umfassen 2,4-Bis-{[4-(2-ethylhexyloxy)-2-hydroxy]-phenyl}-6-(4-methoxyphenyl)-1,3,5-triazin.

Weitere geeignete UV-Filter umfassen 2-Cyano-3,3-diphenylacrylsäure-2-ethylhexylester (z.B. Eusolex® OCR), 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX), 2,4,6-Trianilino-(p-carbo-2'-ethylhexyl-1'-oxi)-1,3,5-triazin (z.B. Uvinul® T 150), 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®), 4,4'-[(6-[4-((1,1-Dimethyl-ethyl)-aminocarbonyl)phenylamino]-1,3,5-triazin-2,4-diyl)diimino]bis(benzoesäure-2-ethylhexylester) (z.B. Uvasorb® HEB), $\alpha$-(Trimethylsilyl)-$\omega$-[trimethylsilyl)oxy]poly[oxy-(dimethyl [und ca. 6% methyl [2-[p-[2,2-bis(ethoxycarbonyl]vinyl]phenoxy]-1-methylene-thyl] und ca. 1,5 % methyl[3-[p-[2,2-bis(ethoxycarbonyl) vinyl)phenoxy)-propenyl) und 0,1 bis 0,4% (methylhydrogen]silylen]] ($n \approx 60$) (CAS-Nr. 207 574-74-1), 2,2'-Methylen-bis-(6-(2H-benzotriazol-2-yl)-4-(1,1,3,3-tetramethylbutyl)phenol) (CAS-Nr. 103 597-45-1), 2,2'-(1,4-Phenylen)bis-(1H-benzimidazol-4,6-disulfonsäure, Mononatriumsalz) (CAS-Nr. 180 898-37-7) und 2,4-bis-{[4-(2-Ethyl-hexyloxy)-2-hydroxyl]-phenyl}-6-(4-methoxy-phenyl)-1,3,5-triazin (CAS-Nr. 103 597-45-, 187 393-00-6).

Bevorzugte UV-Strahlung absorbierende organische Verbindungen sind 3-(4'-Methylbenzyliden)-dl-kampfer, 1-(4-tert-Butylphenyl)-3-(4-methoxyphenyl)propan-1,3-dion, 4-Isopropyl-dibenzoylmethan, 2-Hydroxy-4-methoxybenzophenon, Methoxyzimtsäureoctylester, Methoxyzimtsäureoctylester, 3,3,5-Trimethylcyclohexylsalicylat, 4-(Dimethylamino)-benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Photostabile UV-Strahlung absorbierende organische Verbindungen werden bevorzugt verwendet, wobei photostabile UVA-Filter und UVB-Filter besonders bevorzugt verwendet werden.

Geeignete Substanzen mit antioxidativen und/oder radikalinhibierenden Eigenschaften umfassen z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide wie z.B. D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-,

Butyl- und Lauryl-, Palmitoyl-, Oleyl-, γ-Linoleyl, Cholesteryl- und Glycerylester), Diaurylthiodipropionat, Distearylthiodipropionat, Thiodipropiosäure und deren Derivate (Ester, Ether, Peptide, Lipide, Nukleotide und Nukleoside) sowie Sulfoximinverbindungen (z.B. Buthioninsulfoximine, Homocysteinsulfoximin, Buthioninsulfone, Penta-, Hexa-, Heptathioninsulfoximin), Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Vitamin E und dessen Derivate, Stilbene und deren Derivate (z.B. Stilbenoxid, trans-Stilbenoxid) sowie BHT (2,6-Di-tert-Butyl-4-methyl-phenol).

Bevorzugte Antioxidantien umfassen Flavonoide, Coumaranone, Vitamine und BHT.

Als Flavanoide werden die Glycoside von Flavanonen, Flavonen, 3-Hydroxyflavonen (= Flavanolen), Auronen, Isoflavonen und Rotenoiden aufgefaßt (Römpp Chemie Lexikon, Band 9, 1993). Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavanone sind durch folgende Grundstruktur gekennzeichnet:

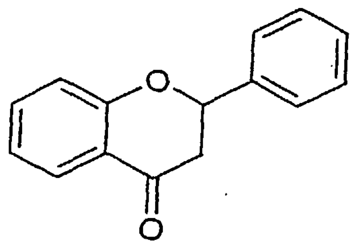

Die Flavone sind durch folgende Grundstruktur gekennzeichnet:

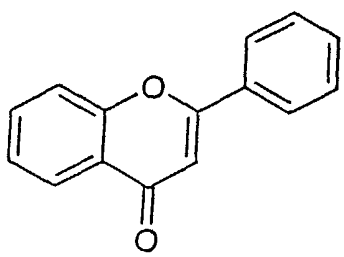

Die 3-Hydroxyflavone (Flavonole) sind durch folgende Grundstruktur gekennzeichnet:

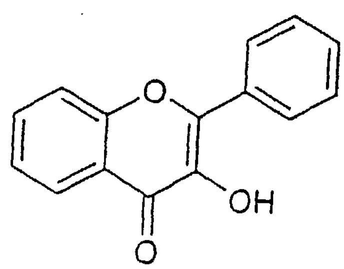

Die Isoflavone sind durch folgende Grundstruktur gekennzeichnet:

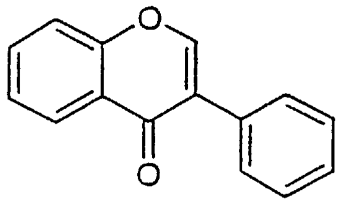

Die Aurone sind durch folgende Grundstruktur gekennzeichnet:

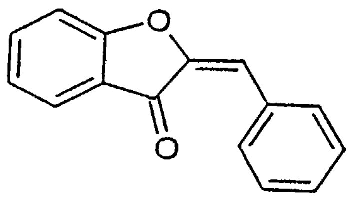

Die Coumaranone sind durch folgende Grundstruktur gekennzeichnet:

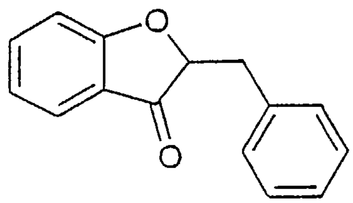

Vorzugsweise werden die Flavonoide und Coumaranone ausgewählt aus den Verbindungen der Formel (1):

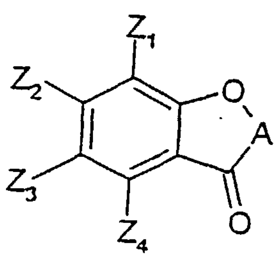

(1)

worin bedeuten:

$Z_1$ bis $Z_4$ jeweils unabhängig voneinander H, OH, Alkoxy, Hydroxyalkoxy, Mono- oder Oligoglycosidreste, wobei die Alkoxy- und Hydroxyalkoxygruppen verzweigt und unverzweigt sein und 1 bis 18 Kohlenstoffatome aufweisen können und wobei an die Hydroxygruppen der genannten Reste auch Sulfat oder Phosphat gebunden sein kann,

A ausgewählt wird aus der Gruppe, bestehend aus den Teilformen (1A), (1 B) und (1C)

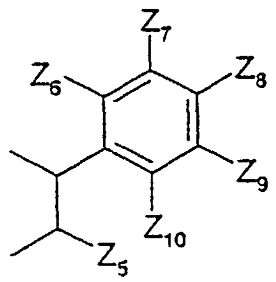

(1A)

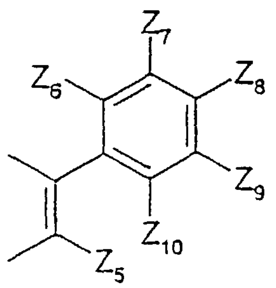

(1B)

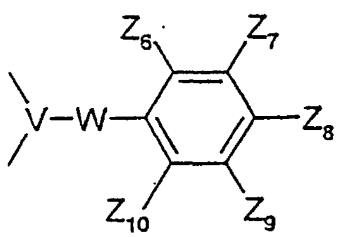

(1C)

$Z_5$ H, OH oder OR,

R einen Mono- oder Oligoglycosidrest,

$Z_6$ bis $Z_{10}$ die Bedeutung der Reste $Z_1$ bis $Z_4$ besitzen, und

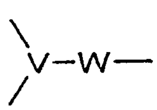 bedeutet 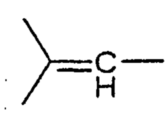 oder 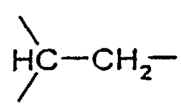

Die Alkoxygruppen sind vorzugsweise linear und besitzen 1 bis 12, vorzugsweise 1 bis 8 Kohlenstoffatome. Diese Gruppen entsprechen somit der Formel $-O-(CH_2)_m-H$, wobei m 1,2,3,4,5,6,7 oder 8 und insbesondere 1 bis 5 bedeutet.

Die Hydroxyalkoxygruppen sind vorzugsweise linear und besitzen 2 bis 12, vorzugsweise 2 bis 8 Kohlenstoffatome. Diese Gruppen entsprechen somit der Formel $-O-(CH_2)_n-OH$, wobei n 2,3,4,5,6,7 oder 8, insbesondere 2 bis 5 und besonders bevorzugt 2 bedeutet.

Die Mono- und Oligoglycosidreste sind vorzugsweise aus 1 bis 3 Glycosideinheiten aufgebaut. Vorzugsweise werden diese Einheiten ausgewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl, sind gegebenenfalls vorteilhaft zu verwenden. Es kann auch erfindungsgemäß vorteilhaft sein, Pentosylreste zu verwenden.

In einer bevorzugten Ausführungsform besitzen

| | |
|---|---|
| $Z_1$ und $Z_3$ | die Bedeutung H, |
| $Z_2$ und $Z_4$ | eine andere Bedeutung als H, insbesondere bedeuten sie OH, Methoxy, Ethoxy oder 2-Hydroxyethoxy, |
| $Z_5$ | die Bedeutung H, OH oder einen Glycosidrest, der aus 1 bis 3, vorzugsweise aus 1 oder 2, Glycosideinheiten aufgebaut ist. |
| $Z_6$, $Z_9$ und $Z_{10}$ | die Bedeutung H, und |
| $Z_7$ und $Z_8$ | eine andere Bedeutung als H, insbesondere bedeuten sie OH, Methoxy, Ethoxy oder 2-Hydroxyethoxy. |

Besonders bevorzugte Verbindungen werden durch die folgende allgemeine Formel dargestellt:

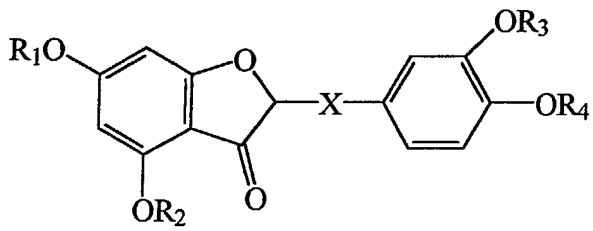

wobei - X - steht für eine Einfachbindung, - $CH_2$- oder = CH-, und

$R^1$, $R^2$, $R^3$ und $R^4$ gleich oder verschieden sein können, und unabhängig voneinander stehen für

H,
geradkettige oder verzweigte $C_1$- bis $C_{12}$-Alkylgruppen und/oder

- Alkylcarbonylgruppen
  geradkettige oder verzweigte $C_3$- bis $C_{12}$-Alkenylgruppen und/oder
- Alkenylcarbonylgruppen

geradkettige oder verzweigte $C_1$- bis $C_{12}$-Hydroxyalkyl- und/oder-Hydroxyalkylcarbonylgruppen, wobei die Hydroxygruppe an ein primäres oder sekundäres Kohlenstoffatom der Kette gebunden sein kann und weiter die Alkylkette auch durch Sauerstoff unterbrochen sein kann,

$C_3$- bis $C_{10}$-Cycloalkyl- und/oder -Cycloalkylcarbonylgruppen und

$C_3$- bis $C_{12}$-Cycloalkenyl- und/oder -Cycloalkenylcarbonylgruppen, wobei die Ringe auch durch $-(CH_2)_n$-Gruppen mit n = 1 bis 3 überbrückt sein können,

Arylgruppen und/oder Arylcarbonylgruppen
Heteroarylgruppen und/oder Heteroarylcarbonylgruppen

wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,

Mono- oder Oligoglycosidreste,

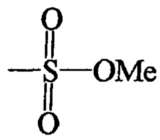

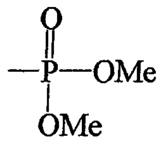

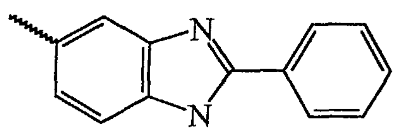

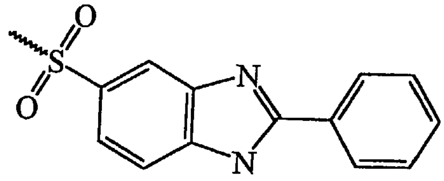

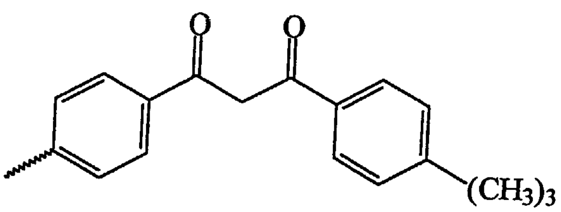

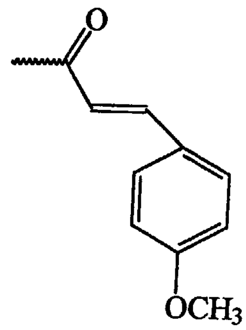

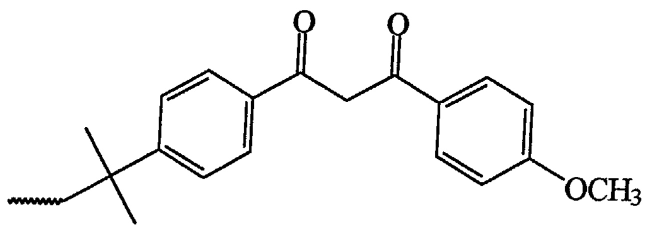

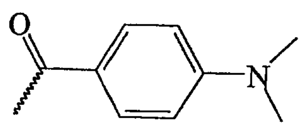

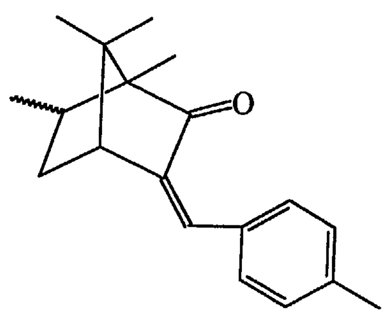

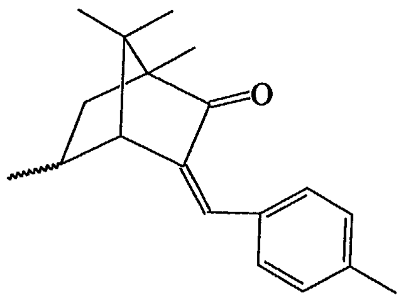

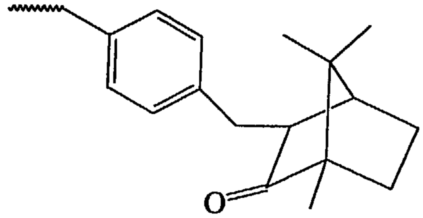

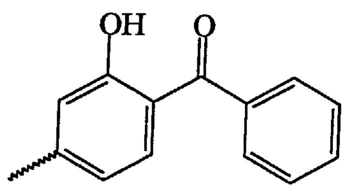

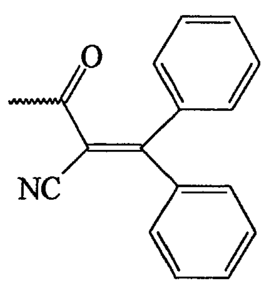

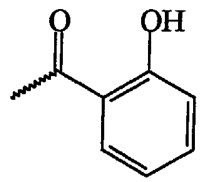

wobei $R^5$ steht für tert.-Butyl oder Isopropyl und

Me steht für ein Proton oder ein Alkalimetallion, insbesondere ein Kaliumion.

Die Reste können also als Ether oder als Ester an den Grundkörper gebunden sein.

In einer weiteren bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin) sowie Trishydroxyethylluteolin (Troxeluteolin).

Unter den Flavonoiden sind insbesondere Rutin und Troxerutin bevorzugt. Besonders bevorzugt ist Troxerutin.

Unter den Coumaranonen ist 4,6,3',4'-Tetrahydroxybenzylcoumaranon-3 bevorzugt.

Geeignete Repellentien umfassen Amide und deren Derivate.

Geeignete Konservierungsmittel umfassen Benzoesäure und deren Salze (wie z.B. Natriumbenzoat), Methylparaben, Ethylparaben, Propylparaben, Sorbinsäure und deren Salze (wie z.B. Kaliumsorbat) sowie Salicylsäure und deren Salze (wie z.B. Natriumsalicylat).

Der erfindungsgemäß verwendete Wirkstoff kann ebenfalls eine entzündungshemmende Substanz sein.

Der erfindungsgemäß verwendete Wirkstoff ist bevorzugt öl- und/oder wasserlöslich und wird durch die kovalente Bindung an das anorganische Pigment in einen Zustand überführt, in dem er nicht mehr in die Haut eindringen bzw. penetrieren kann.

Erfindungsgemäß ist es bevorzugt, dass die Spacer-Gruppe ein reaktives Metallzentrum umfasst.

Das erfindungsgemäße Konjugat wird vorzugsweise durch die folgende Formel (I) dargestellt:

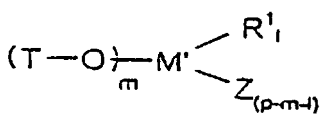

(I)

worin bedeuten:

T ein Oxid eines Elements, ausgewählt aus der Gruppe Si, B, Al, Fe, Sn, Ti und Zr, oder ein Mischoxid dieser Elemente, im Kern des Pigments befindlich;

M' ein reaktives Metallzentrum, umfassend ein Element der Gruppen 3A, 4A, 3B, 4B, 5B oder 6B des Periodensystems der Elemente;

$R^1$ die kovalent gebundene Wirkstoffgruppe;

Z gleich oder unterschiedlich sein kann und H, OH, ein Halogenatom, ausgewählt aus F, Cl, Br und/oder einen organischen Rest, ausgewählt aus A, OA, ACOO, $NA_2$, SA, gesättigtes oder ungesättigtes Cycloalkyl mit bis zu 6 C-Atomen, heterocyclischer oder aromatischer Rest Ar,

mit

y 0,1,2,3,4

A geradkettiger, verzweigter, gesättigter oder ungesättigter Alkylrest mit 1 bis 8 C-Atomen

und

Ar substituiertes oder unsubstituiertes Phenyl, Naphthyl, Pyridyl, Pyrimidinyl, Thiophenyl, Furanyl,

wobei die Substituenten A, OA, COOH, COOA, Cl, F sein können, bedeutet;

p die maximal mögliche Bindungszahl von M';
m 1,2 oder 3 und
l ≤ (p-m).

Weitere bevorzugte Beispiele für T umfassen die Oxide und Mischoxide, die zuvor als bevorzugte anorganische Pigmente und Mischpigmente genannt wurden.
Mit anderen Worten, das erfindungsgemäße Konjugat der Formel (I) wird insbesondere durch die folgenden Formeln (II), (III) und (IV) dargestellt:

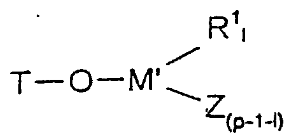

(II)

(III)

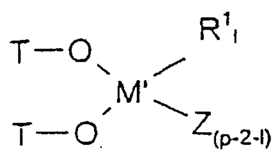

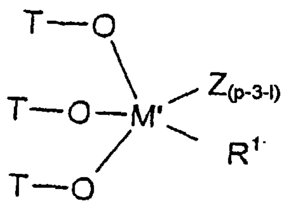

(IV)

In diesem Fall wird die Spacergruppe durch $-(O)_m$-M' dargestellt.
Vorzugsweise stellt T ein Oxid eines Elements, ausgewählt aus der Gruppe Si, B, Al, Fe, Sn, Ti und Zr, oder ein Mischoxid dieser Elemente dar.
M' ist vorzugsweise Al, Si, Ti oder Zr.
Z ist vorzugsweise H, OH, ein Halogenatom, A, OA oder ACOO.
Insbesondere wird das erfindungsgemäße Konjugat vorzugsweise durch die Formel (II) oder (III) dargestellt.
Bevorzugte Beispiele für $R^1$ umfassen:

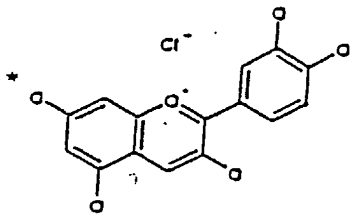

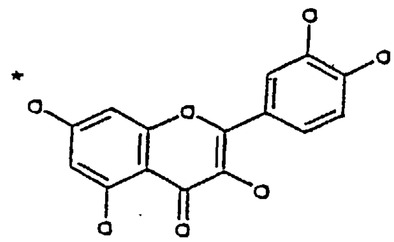

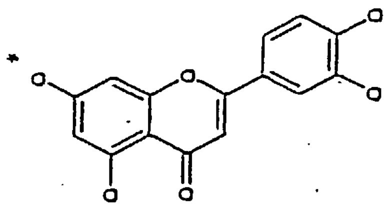

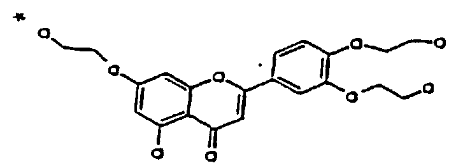

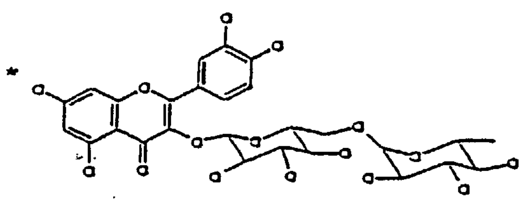

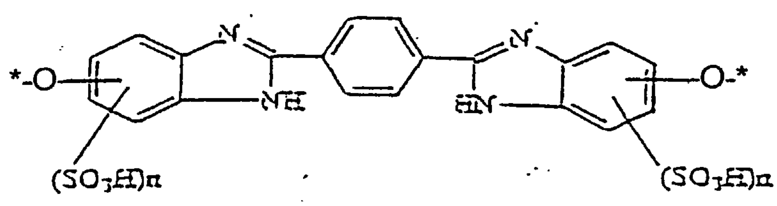

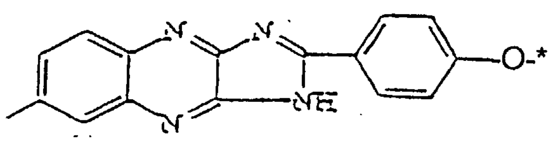

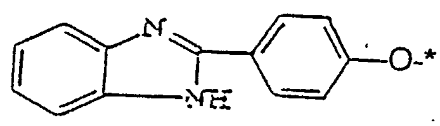

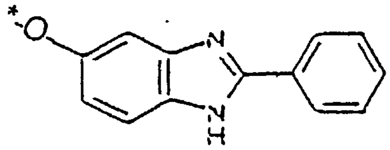

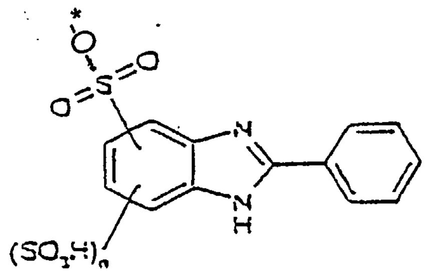

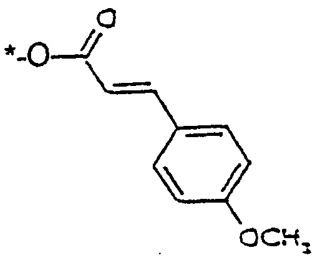

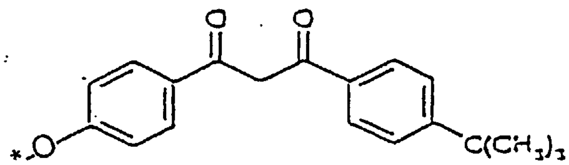

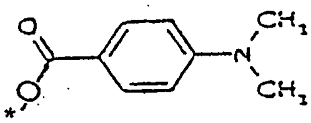

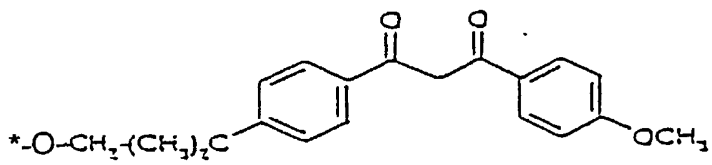

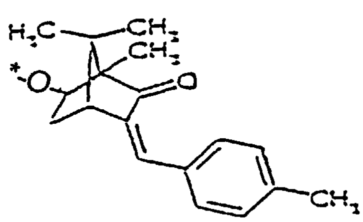

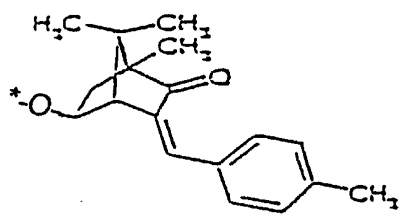

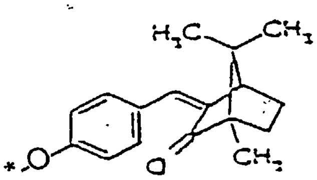

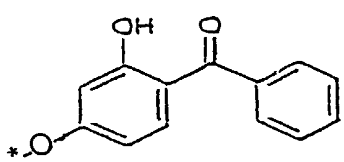

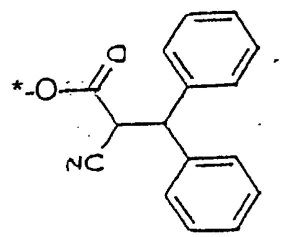

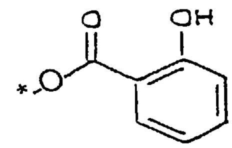

worin n 0, 1 oder 2 bedeutet, und * ist die Bindung zur Spacer-Gruppe.

Bei den zuvor genannten Verbindungen erfolgt die Bindung zur Spacer-Gruppe über ein Sauerstoffatom. Die Bindung zur Spacer-Gruppe kann jedoch auch über eine andere Verbindungsgruppe erfolgen, wie z.B. über die Gruppen -NH-, $-OCH_2O-$ oder $-OCH_2CH_2O-$.

Bevorzugte Beispiele des erfindungsgemäßen Konjugats umfassen:

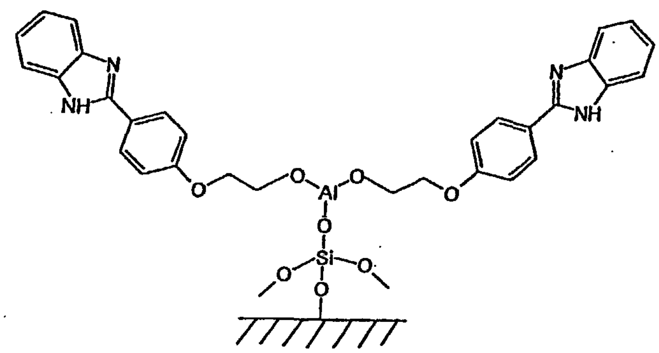

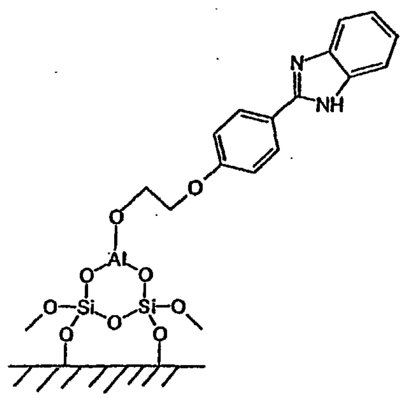

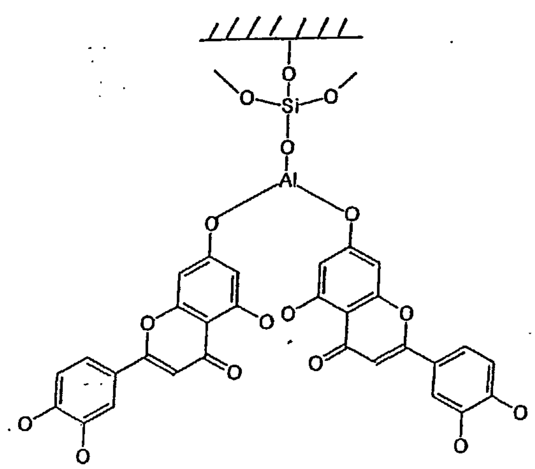

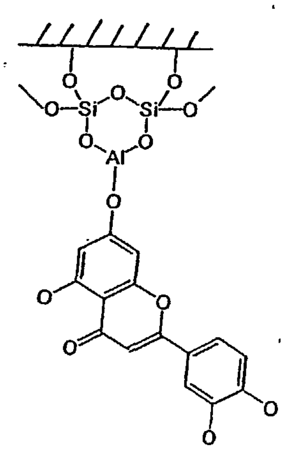

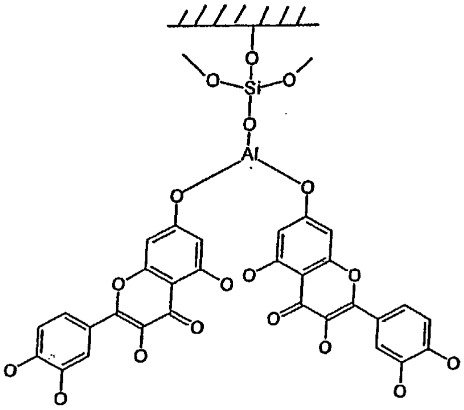

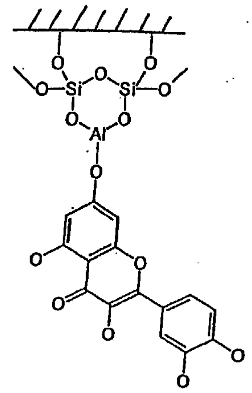

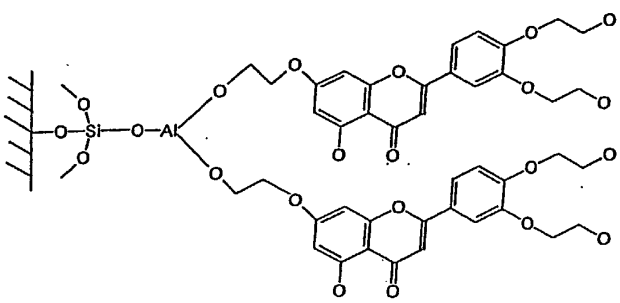

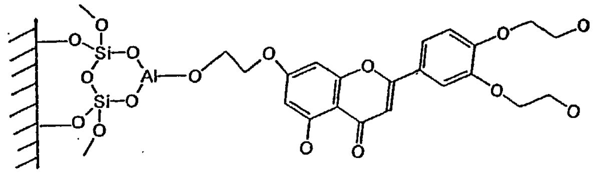

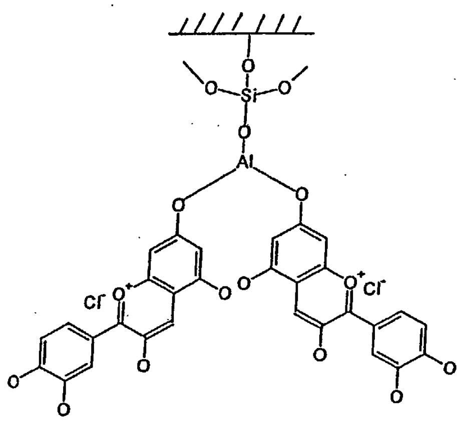

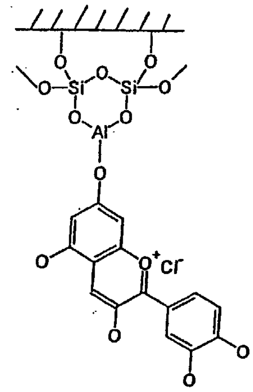

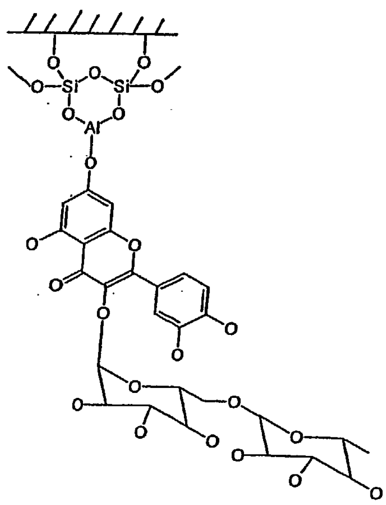

oder

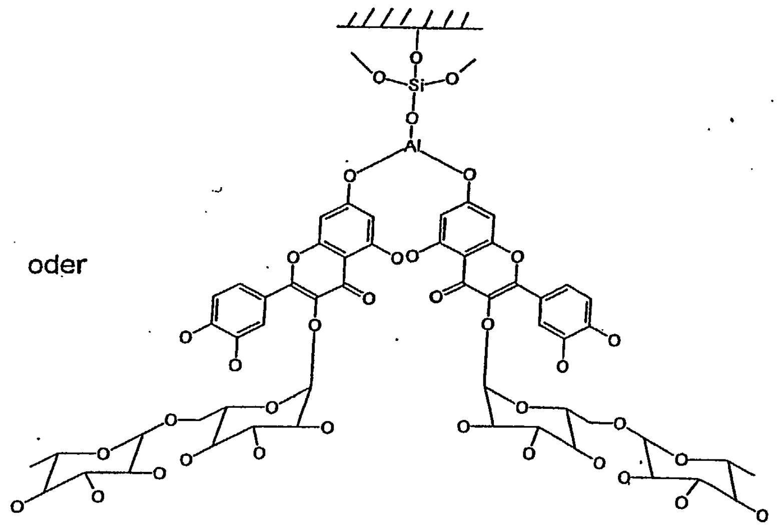

wobei die Strukturen

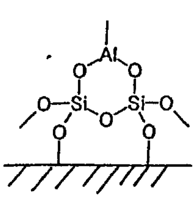

und

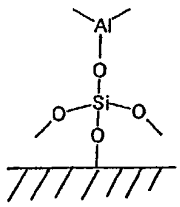

den anorganischen Träger (Festkörper), in diesem Fall ein Siliciumdioxid, das über eine Aluminiumorganylverbindung zur Konjugatbildung des UV-Filters benutzt wird, darstellen. Die Konjugate sind jedoch nicht auf diese Strukturen beschränkt.

Bevorzugte Konjugate sind solche, in denen mindestens zwei Wirkstoffmoleküle an ein reaktives Metallzentrum M' gebunden sind.

Zur Herstellung des erfindungsgemäßen Konjugats kann der Wirkstoff auch über eine organische Verbindungsgruppe kovalent an den anorganischen Träger gebunden. Im Rahmen der vorliegenden Erfindung wird unter dem Begriff "organische Verbindungsgruppe" eine monomere oder oligomere Verbindungsgruppe, jedoch keine polymere Gruppe, verstanden, wie z.B. $-O(CH_2CH_2O)_n-$, worin n eine ganze Zahl im Bereich von 1 bis 20 ist. n ist bevorzugt 1 oder 2.

Das erfindungsgemäße Konjugat kann hergestellt werden, indem zunächst das anorganische Pigment bzw. Substrat mit einer metallorganischen Verbindung zu einem Trägermaterial mit einer reaktiven Oberfläche modifiziert wird. Diese reaktive Oberfläche reagiert dann je nach ihrer chemischen Natur mit reaktiven Gruppen des Wirkstoffes. Diese reaktiven Gruppen können z.B. aktiver Wasserstoff (aus kohlenstoff- oder heteroatomhaltigen Gruppen) oder ein Metallderivat eines Nucleophils sein.

Ein typisches Verfahren wird z.B. in der DE-A-19802753 beschrieben. Darin wird das anorganische Pigment, das an der Oberfläche OH- oder OM-Gruppen besitzt, worin M einem Alkali- oder Erdalkaliatomäquivalent entspricht, mit einem Metallderivat umgesetzt. Das Metallderivat ist ein Metallhalogenid, -organyl oder -hydrid oder ein Gemisch davon, worin das Metallion wie M' definiert ist, Halogen wie vorstehend definiert ist und -organyl wie der organische Rest unter Z definiert ist.

Als Metallhalogene oder -hydride kommen Verbindungen in Frage, bei denen ein oder mehrere der organischen Reste durch das Halogenatom und/oder durch H ersetzt sein kann.

Demzufolge wird das Metallderivat durch die Formel $M'(Z)_p$ dargestellt, mit der Maßgabe, dass das Metallderivat mindestens eine Fluchtgruppe, d.h. ein Halogenatom oder einen Alkoxy-, Acyloxy- oder Organylsulfatrest, enthält. Die erhaltenen oberflächenreaktiven anorganischen Pigmente werden folglich durch die allgemeine Formel (V) bzw. die Formeln (VI), (VII) oder (VIII) dargestellt:

$$(T\text{-}O)_m\text{-}M'\text{-}Z_{(p-m)} \qquad (V)$$

$$T-O-M'-\left[Z\right]_{p-1} \qquad (VI)$$

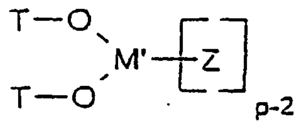

(VII)

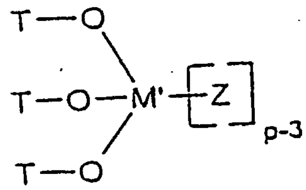

(VIII)

Dieses Zwischenprodukt kann danach kovalent an den Wirkstoffrest $R^1$ gebunden werden. Dies geschieht üblicherweise durch nucleophile Substitution.

Das vorstehend genannte Zwischenprodukt wird folglich mit ionischen Nucleophilen (IX) oder mit Nucleophilen (X), die mit Wasserstoff substituiert sind, umgesetzt:

$$R^{1\ominus} \quad \text{(IX)}$$

$$R^1 - H \quad \text{(X)}$$

Das erfindungsgemäße Konjugat kann damit nach folgendem Reaktionsschema erhalten werden:

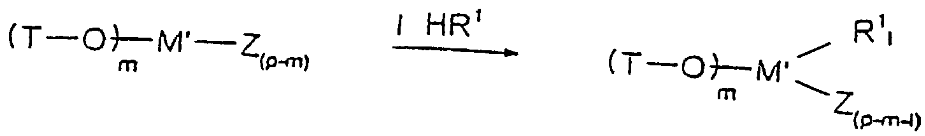


(V) (I)

Insbesondere können die folgenden Umsetzungen erfolgen:

(VI)

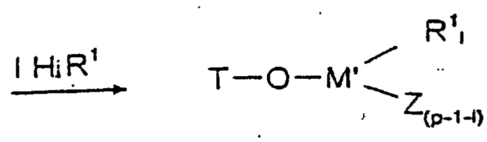


(II)

mit l = 1 - 3

(VII) 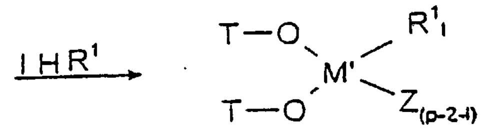


(III)

mit l = 1 - 2

und

(VIII) 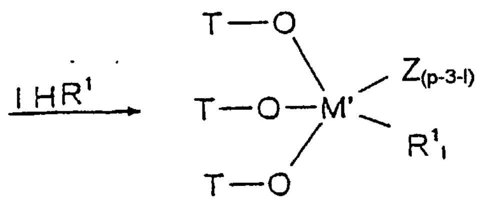


(IV)

mit l = 1

Die Synthese des erfindungsgemäßen Konjugats kann in einem inerten Lösungsmittel, das in Abhängigkeit von den eingesetzten Ausgangsmaterialien ausgewählt wird, oder durch Aufdampfen des Wirkstoffes auf die reaktive Oberfläche des Pigmentsubstrats erfolgen.

Die Anzahl der reaktiven Gruppen, wie Hydroxygruppen, an der Oberfläche des anorganischen Pigments kann mit dem Verfahren, das in der DE-A-198 02 753 beschrieben ist, erhöht werden.

Anorganische Trägermaterialien weisen je nach chemischem Aufbau eine mehr oder weniger große Zahl an reaktiven OH-Gruppen an der Oberfläche auf, die eine chemische Bindung eingehen können. Diese Zahl beträgt z.B. für vollständig hydroxylierte $SiO_2$-Monosphären ca. 4,4 bis 8,5 pro $nm^2$ (H. P. Boehm, Angew. Chem. 78, 617 (1966)). Bestätigt wurden diese Werte durch J. Kratochvila et al., Journal of Non-Crystalline Solids 143, 14-20 (1992). Bei einem Bindungsabstand von ca. 0,16 nm für die Si-O-Bindung und einem Winkel von 150° für den Si-O-Si-Winkel befinden sich an der Oberfläche der $SiO_2$-Monosphären ca. 13 Si-Atome pro $nm^2$. Das bedeutet, dass in der oberflächigen Monoschicht maximal 13 Si-OH-Gruppen auftreten bei zusätzlicher 3-facher Valenzbindung des Si über die oxidischen Sauerstoffbrücken. In der Regel sind bei $SiO_2$-Monosphären, die bei Raumtemperatur getrocknet wurden, jedoch nur 4 Si-OH-Gruppen zu erwarten (vergl. Boehm und Kratochvila).

Um auf den $SiO_2$-Monosphären pro Gewichtseinheit eine möglichst große Anzahl aktiver Si-OH-Gruppen zu erhalten, können die Oberflächen der $SiO_2$-Monosphären in Form von Poren, Klüften und/oder durch möglichst kleine Partikeldurchmesser vergrößert werden. Eine Erhöhung der Anzahl der Si-OH-Gruppen durch Sättigung mit Wasser oder Wasserdampf führt zu keiner befriedigenden Lösung, denn das zusätzlich an der Oberfläche adsorbierte Wasser bewirkt bei den meisten chemisorptiven Belegungen der Oberfläche mit Liganden deren Hydrolyse. Starkes Trocknen wiederum führt gleichzeitig zu einer Abnahme der Anzahl der Si-OH-Gruppen auf bis unter 2 pro $nm^2$.

Entsprechend dem Verfahren, das in der DE-A-198 02 753 beschrieben wird, wird z.B. ein Oxid oder Silikat in einem inerten aprotischen Lösungsmittel, wie z.B. THF, DMF, Cyclohexan oder Toluol, mit einem stark basischen Reagenz, wie z.B. einem Alkali- oder Erdalkalihydroxid, einem Hydrid oder einem Alkoholat, behandelt, um die oberflächlichen Oxidbindungen des Oxids oder Silikats zu spalten, und das behandelte Oxid oder Silikat wird dann mit einer anorganischen oder organischen Säure behandelt, um zusätzliche Hydroxygruppen zu erzeugen.

Wenn die erfindungsgemäß verwendeten anorganischen Pigmente entsprechend diesem Verfahren behandelt werden, kann die Bindungsdichte der Wirkstoffe an der Oberfläche des anorganischen Pigments erhöht werden.

Die Partikelgröße des erfindungsgemäßen Konjugats ist nicht auf eine bestimmte Partikelgröße beschränkt; sie liegt jedoch gewöhnlich im Bereich von 1 nm bis 250 µm, bevorzugt im Bereich von 1 nm bis 1 µm und besonders bevorzugt im Bereich von 5 nm bis 100 nm, um eine optimale Verteilung auf der Haut zu gewährleisten.

Das erfindungsgemäße Konjugat kann nach dessen Herstellung getrocknet und dann in die dermatologische

oder kosmetische Zusammensetzung eingebracht werden, oder es kann in Form einer Dispersion, z.B. dispergiert in einem kosmetischen Öl oder flüssigen Lichtfilter, in die dermatologische oder kosmetische Zusammensetzung eingebracht werden.

Verwendbare Ölkomponenten umfassen natürliche und synthetische Substanzen, wie z.B. Paraffinöl, Glycerylstearat, Isopropylmyristat, Diisorpopyladipat, 2-Ethylhexansäure-acetylstearylester, hydriertes Polyisobuten, Vaseline, Caprylsäure/Caprinsäure-Triglyceride, mikrokristallines Wachs, Lanolin, Mineralöle, Mineralwachse, Ester von Fettsäuren mit Alkoholen, wie z.B. Isopropanol, Propylenglykol oder Glycerin, Alkylbenzoate, Silikonöle, wie z.B. Dimethylpolysiloxane, Diethylpolysiloxane oder Diphenylpolysiloxane, Stearinsäure, Lichtfilter, die in flüssiger Form vorliegen, sowie Vitamin K1. Bevorzugte Beispiele umfassen Ethylbutylacetylaminopropionat (IR 3535™), Butylenglycoldicaprylat/dicaprinat (Miglyol 8810), Propylenglycoldicaprylat/dicaprinat), C12-15 Alkylbenzoate, Isopropylmyristat, Caprylsäure/-Caprinsäure-Triglyceride, Octylpalmitat, Mandelöl, Avocadoöl, Jojobaöl, Isostearylisostearat, Octyldodecanol, Dibutyladipat (Cetiol B), Cocossäureglyceride (Myritol 331), Dicaprilylether (Cetiol OE), Isostearylneopentanoat (Ceraphyl 375), C12-15 Alkyllactate (Ceraphyl 41), Dioctylmalat (Ceraphyl 45), sowie die flüssigen Lichtfilter Eusolex® 2292, Eusolex® OCR, Eusolex® 6007, Eusolex® HMS und Eusolex® OS.

Die erfindungsgemäße dermatologische oder kosmetische Zusammensetzung enthält das zuvor beschriebene Konjugat oder eine Kombination der zuvor beschriebenen Konjugate und mindestens einen kosmetisch, pharmazeutisch und/oder dermatologisch verträglichen Träger und/oder Hilfsstoff. Die dermatologische oder kosmetische Zusammensetzung kann das erfindungsgemäße Konjugat natürlich auch in Kombination mit anderen Wirkstoffen, wie z.B. organischen oder anorganischen UVA-und UVB-Filtern, IR- oder VIS-Filtern, oder in Kombination mit Repellentien, die keine Konjugate bilden, wie z.B. N,N-Diethyl-3-methyl-benzamid, 3-[N-n-Butyl-N-acetyl]-aminopropionsäureethylester (IR3535®) oder N,N-Caprylsäurediethylamid (IR790®), oder in Kombination mit Konservierungsmitteln, die keine Konjugate bilden, wie z.B. Benzalkoniumchlorid, Cetylpyridiniumchlorid oder Cetrimoniumchlorid, enthalten. Besonders bevorzugt ist die Kombination mit weiteren UV-Filtern, umfassend die zuvor beschriebenen ungebundenen Lichtfilter, oder deren Gemischen. Dabei liegt das Verhältnis von gebundenen Wirkstoffen (d.h. Wirkstoffe, die an Pigmente gebunden sind) zu ungebundenen Wirkstoffen (d.h. Wirkstoffe, die nicht an Pigmente gebunden sind) bevorzugt im Bereich von 1:10 bis 10:1 und besonders bevorzugt im Bereich von 1:5 bis 5:1. Es können z.B. an Pigmente gebundene Lichtfilter mit ungebundenen Lichtfiltern kombiniert werden.

Die erfindungsgemäße dermatologische oder kosmetische Zusammensetzung enthält das erfindungsgemäße Konjugat, evtl. in Kombination mit weiteren kosmetischen Stoffen, bevorzugt in einer Menge im Bereich von 0,05 bis 30 Gew.-%, besonders bevorzugt in einer Menge im Bereich von 0,5 bis 10 Gew.-% und ganz besonders bevorzugt in einer Menge im Bereich von 0,5 bis 6 Gew.-%, bezogen auf das Gesamtgewicht der dermatologischen oder kosmetischen Zusammensetzung.

Definitionen der kosmetischen Stoffe:

Beispiele für anorganische UV-Filter umfassen gecoatetes Titandioxid (z.B. Eusolex® T-2000 oder Eusolex® T-AQUA), Zinkoxide (z.B. Sachtotec®), Eisenoxide und Ceroxide. Diese anorganischen UV-Filter werden in der Regel in einer Menge von 0,5 bis 10 Gew.%, vorzugsweise 2 bis 5 Gew.%, in die erfindungsgemäßen dermatologischen oder kosmetischen Zusammensetzungen eingearbeitet.

Beispiele für Träger und Hilfsstoffe umfassen Verdickungsmittel, weichmachende Mittel, Befeuchtungsmittel, grenzflächenaktive Mittel, Emulgatoren, Konservierungsmittel, Mittel gegen Schaumbildung, Parfüms, Fette und Wachse, Lanolin, Treibmittel, Stabilisatoren, Konservierungsmittel, Repellentien, Antioxidantien, Bakterizide, Farbstoffe und/oder Pigmente, welche die Formulierung selbst oder die Haut färben, Filmbildner, Geruchsverbesserer, Komplexbildner und andere in der Kosmetik gewöhnlich verwendete Additive.

Als Dispersions- bzw. Solubilisierungsmittel kann ein kosmetisches Öl, ein Wachs oder ein sonstiger Fettkörper, ein Alkohol oder ein Polyol oder Mischungen davon verwendet werden. Zu den besonders bevorzugten Alkoholen oder Polyolen zählen Ethanol, i-Propanol, Propylenglycol, Glycerin und Sorbit.

Als Emulgatoren kommen vorzugsweise bekannte W/O- und daneben auch O/W-Emulgatoren wie z.B. Polyglycerinester, Sorbitanester oder teilveresterte Glyceride in Betracht.

Typische Beispiele für Fette umfassen Glyceride, und als Wachse können z.B. Bienenwachs, Carnaubawachs, Paraffinwachs oder Mikrowachse, gegebenenfalls in Kombination mit hydrophilen Wachsen, verwendet werden.

Als Stabilisatoren können Metallsalze von Fettsäuren, wie z.B. Magnesium-, Aluminium-und/oder Zinkstearat, eingesetzt werden.

Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon.

Verwendbare Filmbildner umfassen Hydrocolloide, wie z.B. Chitosan, mikrokristallines Chitosan oder quaternäres Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich beispielsweise Formaldehydlösungen, p-Hydroxybenzoat oder Sorbinsäure.

Als Perlglanzmittel kommen beispielsweise Glycoldistearinsäureester, wie z.B. Ethylenglycoldistearat, aber auch Fettsäuren und Fettsäuremonoglycolester in Betracht.

Als Farbstoffe können die für kosmetische Zwecke geeigneten und zugelassenen Substanzen verwendet werden, wie sie beispielsweise in der Veröffentlichung "Kosmetische Färbemittel" der Farbstoffkommission der Deutschen Forschungsgemeinschaft, veröffentlicht im Verlag Chemie, Weinheim, 1984, zusammengestellt sind.

Als Antioxidantien können beispielsweise Aminosäuren, Imidazole, Peptide, Carotinoide, α-Hydroxysäuren, ungesättigte Fettsäuren, Vitamin A, C und/oder E und geeignete Derivate dieser Stoffe verwendet werden, sowie Zink und dessen Verbindungen (wie z.B. ZnO, $ZnSO_4$) oder Selen und dessen Verbindungen (wie z.B. Selenmethionin). Bevorzugte Antioxidantien umfassen die zuvor genannten Substanzen mit antioxidativen Eigenschaften, wobei Flavonoide, Coumaranone, Vitamine und BHT bevorzugt sind.

Mischungen von Antioxidantien können ebenfalls in den erfindungsgemäßen dermatologischen und kosmetischen Zusammensetzungen verwendet werden. Bekannte und handelsüblich erhältliche Mischungen umfassen Mischungen, die Lecithin, L-(+)-Ascorbylpaimitat und Zitronen-säure (z.B. Oxynex® AP), natürliche Tocopherole, L-(+)-Ascorbyipalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder BHT, L-(+)-Ascorbylpalmitat und Zitronensäure (z.B. Oxynex® 2004) als aktive Inhaltsstoffe enthalten.

In einer bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten.

Unter den Flavonoiden sind insbesondere Rutin und Troxerutin bevorzugt. Besonders bevorzugt ist Troxerutin.

Unter den Coumaranonen sind 4,6,3',4'-Tetrahydroxybenzylcoumaranon-3 sowie dessen Salze (Sulfat, Phosphat) bevorzugt.

Die erfindungsgemäße dermatologische oder kosmetische Zusammensetzung kann als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen dermatologischen oder kosmetischen Zusammensetzungen enthalten, insbesondere bevorzugt Vitamin-A-Palmitat, Vitamin C, DL-α-Tocopherol, Tocopherol-E-Acetat, Nicotinsäure, Pantothensäure und Biotin.

Gegebenenfalls können die erfindungsgemäßen dermatologischen oder kosmetischen Zusammensetzungen auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie z.B. Dihydroxyaceton oder α-Ketole sowie Erythrolose, sein.

Anwendungsformen der erfindungsgemäßen dermatologischen oder kosmetischen Zusammensetzungen umfassen: Suspensionen, Emulsionen, Fettstifte, Pasten, Salben, Cremes oder Milch (O/W, W/O, O/W/O, W/O/W), Lotionen, Puder, Seifen, tensidhaltige Reinigungspräparate, Öle, Aerosole, Sprays sowie ölig-alkoholische, ölig-wässrige oder wässrig-alkoholische Gele bzw. Lösungen. Weitere Anwendungsformen sind z.B. Sticks, Shampoos und Duschbäder.

Die wässrige Phase der erfindungsgemäßen dermatologischen oder kosmetischen Zusammensetzungen enthält bevorzugt Alkohole, Diole oder Polyole, sowie Ether, vorzugsweise Ethanol, Isopropanol, 1,2-Propandiol, Propylenglykol, Glycerin, Ethylenglykol, Ethylenglykolmonoethyl- oder -monobutylether oder analoge Produkte, ferner ein oder mehrere Verdickungsmittel, wie z.B. Siliciumdioxid, Aluminiumsilikate, Polysaccharide bzw. deren Derivate, z.B. Hyaluronsäure, Xanthangummi, Hydroxypropylemethylcellulose, oder ein Polyacrylat aus der Gruppe der sogenannten Carbopole.

Verwendbare Ölkomponenten umfassen die zuvor genannten Ölkomponenten.

Salben, Pasten, Cremes und Gele können die üblichen Trägerstoffe enthalten, wie z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, wie z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe, wie z.B. Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe, enthalten.

Suspensionen können die üblichen Trägerstoffe, wie z.B flüssige Verdünnungsmittel, z.B. Wasser, Ethanol oder Propylenglykol, Suspendiermittel, wie z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe, enthalten.

Seifen können die üblichen Trägerstoffe, wie z.B. Alkalisalze von Fettsäuren, Salze von Fettsäurehalbestern, Fettsäureeiweißhydrolysate, Isothionate, Lanolin, Fettalkohol, Pflanzenöle, Pflanzenextrakte, Glycerin, Zucker oder Gemische dieser Stoffe, enthalten.

Tensidhaltige Reinigungsprodukte können die üblichen Trägerstoffe, wie z.B. Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe, enthalten.

Gesichts- und Körperöle können die üblichen Trägerstoffe, wie z.B. synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe, enthalten.

Bevorzugte Beispiele für solche Trägerstoffe umfassen die zuvor genannten Ölkomponenten.

In einer bevorzugten Ausführungsform ist die erfindungsgemäße dermatologische oder kosmetische Zusammensetzung eine Emulsion, welche als Schutzcreme oder-milch vorliegt und außer dem erfindungsgemäßen Konjugat Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf der Basis von natürlichen oder synthetischen Ölen und Wachsen, Lanolin, Fettsäureestern, insbesondere Triglyceriden von Fettsäuren, oder ölig-alkoholische Lotionen auf der Basis eines Alkohols, wie Ethanol, oder eines Glycols, wie Propylenglycol, und/oder eines Polyols, wie Glycerin, und Ölen, Wachsen und Fettsäureestem, wie Triglyceriden von Fettsäuren, dar.

Die erfindungsgemäße dermatologische oder kosmetische Zusammensetzung kann auch als alkoholisches Gel vorliegen, welches einen oder mehrere Alkohole oder Polyole, wie Ethanol, Propylenglycol oder Glycerin, und ein Verdickungsmittel, wie Kieselerde, umfaßt. Die ölig-alkoholischen Gele können außerdem ein natürliches oder synthetisches Öl oder Wachs enthalten.

Die festen Stifte können natürliche oder synthetische Wachse und Öle, Fettalkohole, Fettsäuren, Fettsäureester, Lanolin und andere Fettkörper enthalten.

Ist die erfindungsgemäße dermatologische oder kosmetische Zusammensetzung als Aerosol konfektioniert, werden in der Regel die üblichen Treibmittel, wie Alkane, Fluoralkane und Chlorfluoralkane, verwendet.

Weitere typisch kosmetische Anwendungsformen umfassen Lippenstifte, Lippenpflegestifte, Rouge, Puder-, Emulsions- und Wachs-Make up sowie Prä-Sun- und After-Sun-Präparate.

Alle Träger und Hilfsstoffe, die in den erfindungsgemäßen dermatologischen oder kosmetischen Zusammensetzungen verwendet werden können, sind entweder bekannt und handelsüblich erhältlich oder können nach bekannten Verfahren synthetisiert werden.

Die erfindungsgemäße dermatologische oder kosmetische Zusammensetzung kann die zuvor genannten Träger und Hilfsstoffe jeweils in einer Menge im Bereich von 0,001 bis 30 Gew.-%, bevorzugt in einer Menge im Bereich von 0,05 bis 20 Gew.-% und besonders bevorzugt in einer Menge im Bereich von 1 bis 10 Gew.-% enthalten.

Die erfindungsgemäße dermatologische oder kosmetische Zusammensetzung kann mit Hilfe von Verfahren hergestellt werden, die dem Fachmann bekannt sind.

Das erfindungsgemäße Konjugat kann ebenfalls zum Schutz der Haut, insbesondere zum Schutz der Langerhans-Zellen in der Haut, zum Schutz der DNA oder zur Immunprotektion verwendet werden.

Das erfindungsgemäße Konjugat kann auch außerhalb der Kosmetik verwendet werden, z.B. zur Herstellung eines Lackes oder einer Sicherheitsmarkierung. In diesem Fall kann z.B. die Gruppe $R^1$ in der zuvor genannten allgemeinen Formel eine fluoreszierende Gruppe sein, die durch UV-Licht angeregt werden kann.

Die folgenden Beispiele erläutern die Erfindung. Die eingesetzten Ausgangsmaterialien sind entweder handelsüblich erhältlich oder können in bekannter Weise synthetisiert werden.

**Beispiele**

**Beispiel 1**

Herstellung eines Eusolex®232/Monospher® 25-Konjugats

Ein Eusolex®232/Monospher® 25-Konjugat wurde entsprechend dem folgenden Reaktionsschema hergestellt:

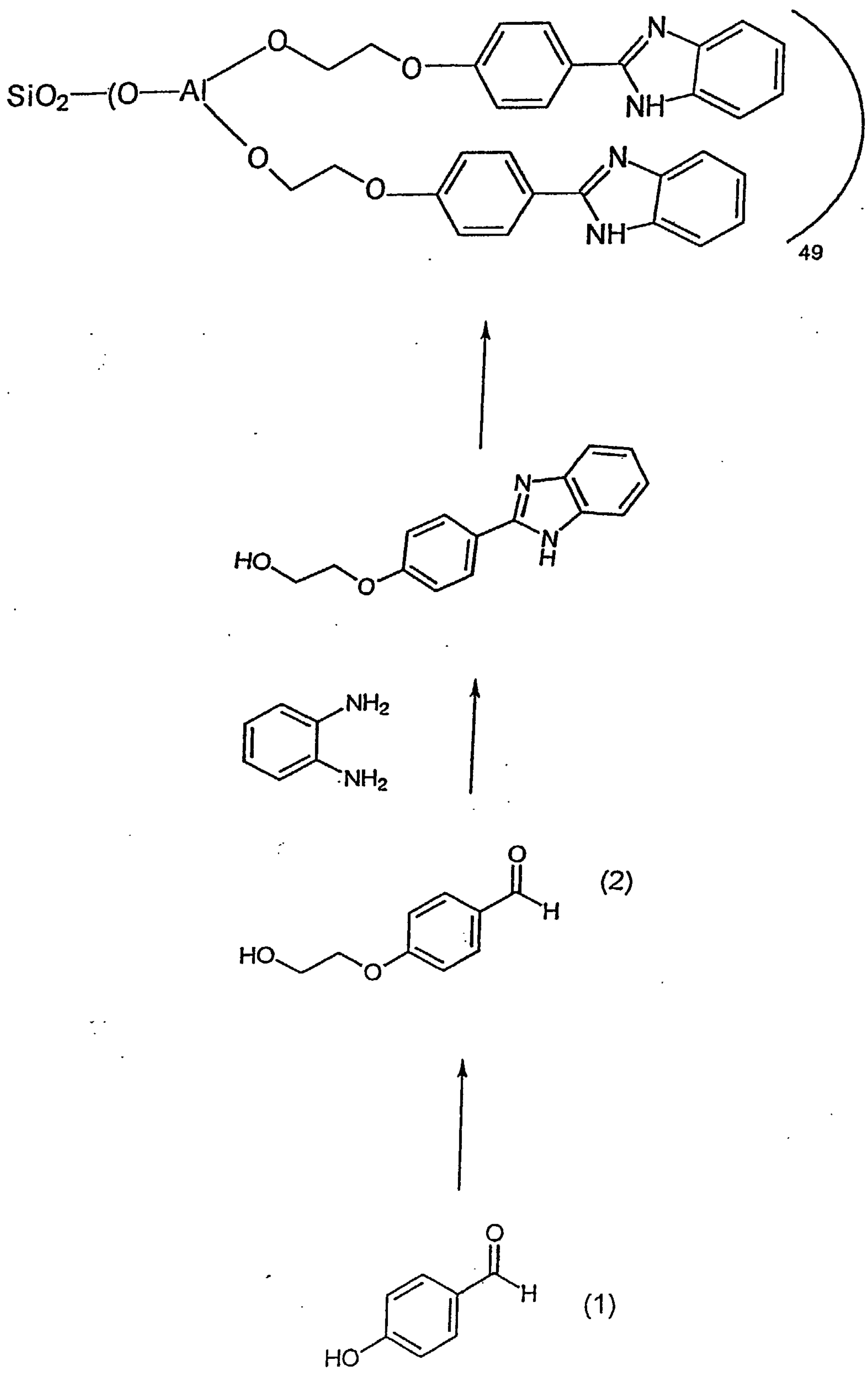

(4)

(3)

(2)

(1)

Herstellung von 4-(2-Hydroxyethoxy)benzaldehyd (2)

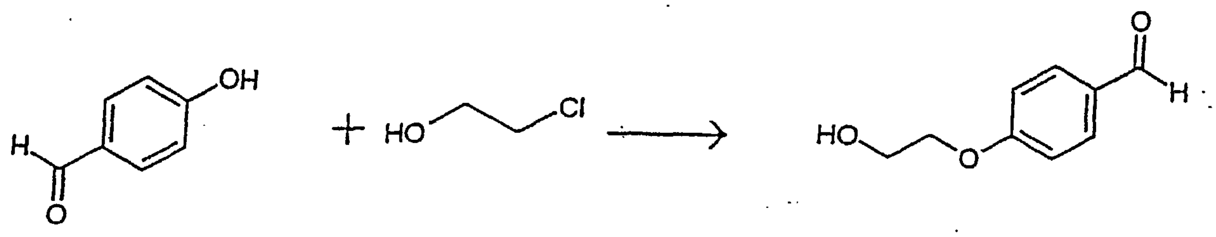

(1) (2)

Die zur Durchführung der Umsetzung verwendete Apparatur wurde mit Stickstoff gespült, um die Luft in der Apparatur zu verdrängen. Dann wurden 12,46 g 4-Hydroxybenzaldehyd (1) in 31,5 g VE-Wasser suspendiert und mit 13,12 g Natronlauge 32%-ig versetzt. Dabei kam es zu einem Temperaturanstieg auf 36°C. Zur Suspension wurden 8,05 g 2-Chlorethanol zudosiert und unter kräftigem Rühren erwärmt, wobei es zur Bildung einer rotbraunen Lösung kam. Das Reaktionsgemisch wurde über Nacht bei 82°C (Badtemperatur 89 bis 90°C) gerührt und es kam zur Bildung einer Emulsion. Die Reaktionszeit betrug 20 Stunden.

Das Reaktionsgemisch wurde auf ca. 40°C abgekühlt und mit 100 g Ethylacetat versetzt. Das Zweiphasengemisch (pH 10 bis 11) wurde mit 17,5 g Natronlauge 32%-ig versetzt, um einen pH-Wert von 13 bis 14 einzustellen. Nach einem Absetzen der Phasen wurden die beiden Phasen getrennt und die wässrige Phase wurde mit 50 g Ethylacetat nachextrahiert. Die vereinten organischen Phasen wurden mit 50 g gesättigter Kochsalzlösung gewaschen, über Watte filtriert und am Rotationsverdampfer azeotrop zu einem braunen Öl konzentriert.

Das Rohprodukt wurde unter leichter Erwärmung in 10 g Toluol/Ethylacetat (1:1) gelöst. Die Aufreinigung erfolgte durch Filtration über eine Fritte (Durchmesser: 13 cm) mit 400 g Kieselgel (Art.7734, Korngröße: 0,063 bis 0,200 mm; Schichtdicke: 6,5 cm). Das

Produkt wurde mit Toluol/Ethylacetat (1:1) eluiert. Die vereinten Fraktionen wurden am Rotationsverdampfer zum Rückstand konzentriert.

Ausbeute:

Rohprodukt: 14,26 g

Endprodukt: 12,88 g.

Herstellung von (3)

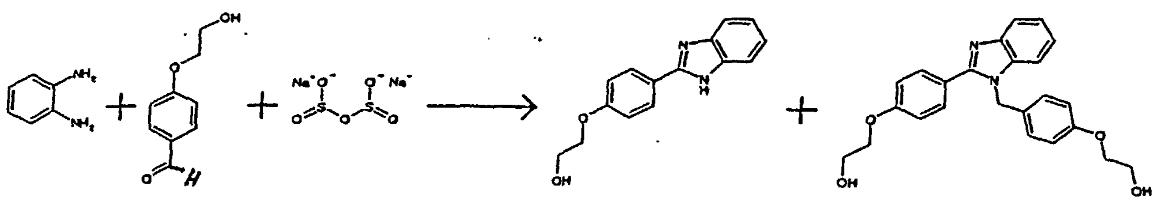

(2) (3)

Die zur Durchführung der Umsetzung verwendete Apparatur wurde mit Stickstoff gespült, um die Luft in der Apparatur zu verdrängen. Dann wurden 8,19 g 1,2-Phenylendiamin vorgelegt und mit einer Lösung von 12,46 g 4-(2-Hydroxyethoxy)-benzaldehyd (2) in 38 g 1-Methyl-2-pyrrolidon versetzt. 21,33 g Natriumdisulfit wurden eingetragen und die Suspension wurde unter kräftigem Rühren erwärmt. Nach 14 Minuten war die Reaktionstemperatur (80°C) höher als die Badtemperatur und stieg in weiteren 2 Minuten noch bis auf 104,5°C. Die Farbe der Suspension wandelte sich mehrmals zwischen gelb, grün und braun. Das Reaktionsgemisch wurde weitere 90 Minuten bei 103°C Innentemperatur (Badtemperatur 103 bis 104°C) gerührt.

Die erhaltene Suspension wurde auf 60°C abgekühlt und in 15 Minuten wurde 135 g VE-Wasser zugetropft. Die dicke Suspension/Emulsion wurde bei einer Temperatur von 40°C langsam in 500 g VE-Wasser laufen gelassen. Die feine Suspension wurde nach 45 Minuten bei Raumtemperatur (27°C) gerührt. Das Rohkristallisat wurde isoliert, portioniert mit 50 bis 70 ml VE-Wasser gewaschen, gut trockengesaugt und über Nacht im Vakuum im Trockenschrank bei 45°C getrocknet.

19 g Rohprodukt wurden in 1000 g Methanol suspendiert und in 45 Minuten auf Rückflusstemperatur (Badtemperatur: 76 bis 80°C) erhitzt. Nach weiteren 30 Minuten wurden innerhalb von 2 Stunden portioniert insgesamt 200 g Methanol zugetropft. Nach 30 Minuten kam es zur vollständigen Lösung und ein geringer Niederschlag wurde bei 65 bis 66°C heiß filtriert und nach DC-Kontrolle verworfen. Die rötliche Lösung wurde am Rotationsverdampfer auf 750 g konzentriert und ein erneut gebildeter Niederschlag wurde abfiltriert. Die Lösung am Rotationsverdampfer wurde weiter eingeengt, wobei ab einem Volumen von ca. 300 ml eine zunehmende Produktkristallisation erkennbar war. Die Suspension wurde auf 110 g konzentriert. Danach wurde das Kristallisat (1. Kristallisat) isoliert, portioniert, mit Methanol gewaschen und trockengesaugt. Aus der Mutterlauge wurde zunächst ein zweites Kristallisat isoliert. Der konzentrierte Mutterlaugenrückstand enthielt ein Gemisch von Produkt und molarem Nebenprodukt. Die Kristallisate wurden im Vakuumtrockenschrank bei 35°C getrocknet.

Ausbeute:

Rohprodukt: 19,3 g
Endprodukt (1. und 2. Kristallisat): 16,02 g

Herstellung von (4)

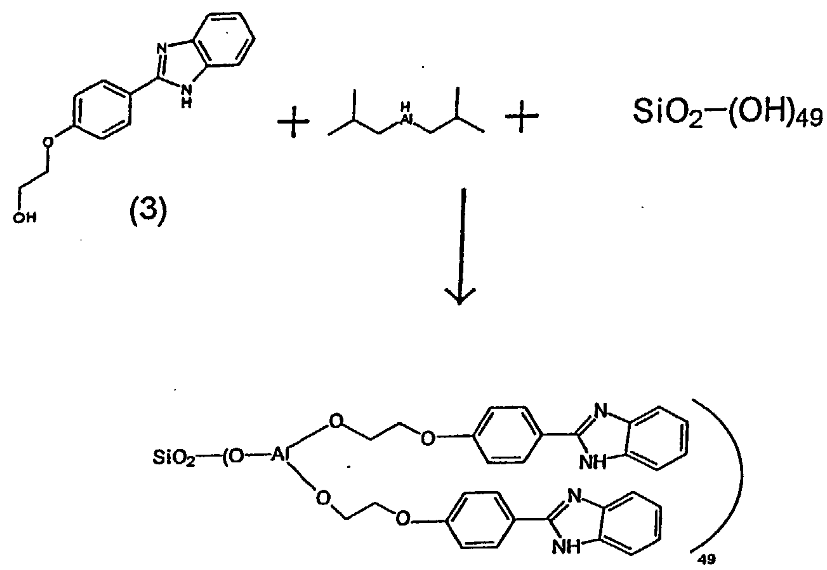


(3)

(4)

a) Erzeugung von Si-ONa-Gruppen

30 g einer Wasserbehandlung und anschließender Trocknung unterworfene Kieselgelkügelchen (Monospheres - Merck KGaA, Darmstadt) mit einem Durchmesser von 500 nm, einer Dichte 2,0, einer berechneten Oberfläche von 6 $m^2$/g, enthaltend Si-OH-Gruppen und Wasser, werden mit 746 mg NaH (3,11 x $10^{-2}$Mol) in 100 ml Tetrahydrofuran bei 30°C versetzt. Dabei tritt Wasserstoffentwicklung auf. Aus diesem Wasserstoffvolumen und der zur Kontrolle des unumgesetzten NaH mit Trifluorsulfonsäure freigesetzten Wasserstoffs ergab sich eine Gesamtmenge von 49 OH-Gruppen pro $nm^2$ (4,9 x $10^{-4}$ Mol OH-Gruppen pro Gramm Kieselgel). Dieser hohe Wert wird dadurch erklärbar, dass Wasser auch in Poren oder Klüften reagiert hat.

Entsprechend diesem Vorversuch werden 30 g der o.g. Kieselkügelchen in 100 ml Tetrahydrofuran mit der berechneten Menge an NaH (353 mg = 1,47 x $10^{-2}$ Mol) zu einem Kieselgel mit 49 Si-ONa-Gruppen pro $nm^2$ umgesetzt, das nachstehend der Einfachheit halber als "$SiO_2$-$(ONa)_{49}$" bezeichnet wird. Die Kügelchen werden abfiltriert und im Vakuum getrocknet. Das Tetrahydrofuranfiltrat reagiert neutral.

b) Umwandlung der SiONa-Gruppen in Si-OH-Gruppen und Substitution der SiOH-Gruppen

$$SiO_2\text{-}(ONa)_{30} \rightarrow SiO_2\text{-}(OH)_{30} \rightarrow SiO_2\text{-}(OAliBu_2)_{30}$$

50 g Kieselgelkügelchen (Monospheres 500, Merck KGaA) von 500 nm Durchmesser mit 30 Si-ONa-Gruppen pro $mm^2$ ($SiO_2$-$ONa_{30}$) werden in 100 ml Tetrahydrofuran mit 1,39 ml (15,8 mMol) $CF_3SO_3H$ zu $SiO_2$-$(OH)_{30}$ umgesetzt. Nach dem Abfiltrieren unter Stickstoff (Druck-Seitzfilter) und Waschen mit Tetrahydrofuran werden die erhaltenen Kügelchen in 100 ml Tetrahydrofuran und mit 15 mMol Diisobutylaluminiumhydrid (12,5 ml einer 1,2 molaren Lösung in Toluol) unter Wasserstoffabspaltung und Bildung von $SiO_2$-$(OAl(iBu)_2)_{30}$ umgesetzt.

c) Einführung organischer Liganden

8 g $SiO_2$-$(OAliBu_2)_{30}$ werden 16 Stunden bei 25°C in 30 ml Tetrahydrofuran mit (3) gerührt. Es wir unter Stickstoff filtriert und mit Tetrahydrofuran gewaschen, um (4) zu erhalten.

**Beispiel 2**

Herstellung eines Cyanidin/Monospher® 500-Konjugats

Ein Cyanidin/Monospher® 500-Konjugat wurde in der folgenden Weise hergestellt.

Die zur Durchführung der Umsetzung verwendete Apparatur wurde mit Stickstoff gespült, um die Luft in der Apparatur zu verdrängen. Dann wurde mit einer Aluminiumverbindung (Di-iso-Butyl-Al) belegtes Monospher® 500 (30 Gruppen pro $nm^2$) in die Apparatur eingebracht und mit THF angeschlämmt, um eine 10%ige Dispersion herzustellen. Danach wurde die folgende Verbindung in die Dispersion eingebracht:

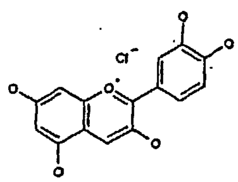

Die erhaltene dunkelrote Reaktionslösung wurde über Nacht bei Raumtemperatur unter Stickstoffatmosphäre gerührt.

Am nächsten Tag wurde die dunkelrote Reaktionslösung über ein Druckfilter abfiltriert. Der Filterrückstand wurde dreimal mit jeweils 20 ml THF und/oder Pyridin nachgewaschen. Der Nutschkuchen, ein violettes feines Pulver, wurde über Nacht im Vakuumtrockenschrank bei Raumtemperatur und 150 mbar getrocknet.

Es konnte nicht abschließend geklärt werden, welche der phenolischen Gruppen der zuvor angegebenen Verbindung reagiert. Es ist auch möglich, dass zwei benachbarte OH-Gruppen mit den beiden Isobutylgruppen eines Aluminiumatoms reagieren.

Parallel zu dem Versuch wurde noch ein Ansatz über Nacht gerührt, wobei das mit einer Aluminiumverbindung (Di-iso-Butyl-Al) belegte Monospher® 500 durch nicht belegtes Monospher® 500 ersetzt wurde. Das Anthocyanidin wurde beim Nachwaschen vollständig vom Monospher® 500 gewaschen, so dass ein weißes Pulver zurückblieb.

**Beispiel 3**

Herstellung eines Quercetin/Monospher® 500-Konjugats

Ein Quercetin/Monospher® 500-Konjugat wurde in der folgenden Weise hergestellt.

Die zur Durchführung der Umsetzung verwendete Apparatur wurde mit Stickstoff gespült, um die Luft in der Apparatur zu verdrängen. Dann wurde mit einer Aluminiumverbindung (Di-iso-Butyl-Al) belegtes Monospher® 500 in die Apparatur eingebracht und mit THF angeschlämmt, um eine 10%ige Dispersion herzustellen. Danach wurde die folgende Verbindung in die Dispersion eingebracht:

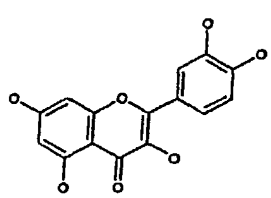

Die erhaltene gelbe Reaktionslösung wurde über Nacht bei Raumtemperatur unter Stickstoffatmosphäre gerührt.

Am nächsten Tag wurde die gelbe Reaktionslösung über ein Druckfilter abfiltriert. Der Filterrückstand wurde dreimal mit jeweils 20 ml THF und/oder Pyridin. Der Nutschkuchen, ein gelbes feines Pulver, wurde über Nacht im Vakuumtrockenschrank bei Raumtemperatur und 150 mbar getrocknet.

Auch in diesem Fall konnte nicht abschließend geklärt werden,, welche der phenolischen Gruppen der zuvor angegebenen Verbindung reagiert.

Parallel zu dem Versuch wurde noch ein Ansatz über Nacht gerührt, wobei das mit einer Aluminiumverbindung (Di-iso-Butyl-Al) belegte Monospher® 500 durch nicht belegtes Monospher® 500 ersetzt wurde. Das Quercetin wurde beim Nachwaschen vollständig vom Monospher® 500 gewaschen, so dass ein weißes Pulver zurückblieb.

**Beispiel 4**

Herstellung eines Luteolin/Monospher® 500-Konjugats

Ein Luteolin/Monospher® 500-Konjugat wurde in der folgenden Weise hergestellt.
Die zur Durchführung der Umsetzung verwendete Apparatur wurde mit Argon gespült, um die Luft in der Apparatur zu verdrängen. Dann wurde mit einer Aluminiumverbindung (Di-iso-Butyl-Al) belegtes Monospher® 500 in die Apparatur eingebracht und mit THF angeschlämmt, um eine 10%ige Dispersion zu erhalten. Danach wurde die folgende Verbindung in die Dispersion eingebracht:

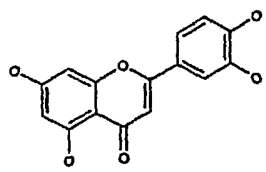

Die erhaltene gelbe Reaktionslösung wurde über Nacht bei Raumtemperatur unter Argonatmosphäre gerührt.
Am nächsten Tag wurde die gelbe Reaktionslösung über ein Druckfilter abfiltriert. Der Filterrückstand wurde dreimal mit je 20 ml THF und/oder Pyridin nachgewaschen. Der Nutschkuchen, ein hellgelbes feines Pulver, wurde über Nacht im Vakuumtrockenschrank bei Raumtemperatur und 150 mbar getrocknet.

**Beispiel 5**

Herstellung eines Triethoxyluteolin/Monospher® 500-Konjugats

Ein Triethoxyluteolin/Monospher® 500-Konjugat wurde in der folgenden Weise hergestellt.
Die zur Durchführung der Umsetzung verwendete Apparatur wurde mit Argon gespült, um die Luft in der Apparatur zu verdrängen. Dann wurde mit einer Afuminiumverbindung (Di-iso-Butyl-Al) belegtes Monospher® 500 in die Apparatur eingebracht und mit THF angeschlämmt, um eine 10%ige Dispersion zu erhalten. Danach wurde die folgende Verbindung in die Dispersion eingebracht:

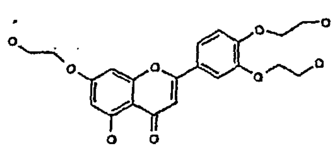

Die erhaltene gelbe Reaktionslösung wurde über Nacht bei Raumtemperatur unter Argonatmosphäre gerührt.
Am nächsten Tag wurde die gelbe Reaktionslösung über ein Druckfilter abfiltriert. Der Filterrückstand wurde dreimal mit je 20 ml THF und/oder Pyridin nachgewaschen. Der Nutschkuchen, ein hellgelbes feines Pulver, wurde über Nacht im Vakuumtrockenschrank bei Raumtemperatur und 150 mbar getrocknet.

**Beispiel 6**

Herstellung eines Rutin/Monospher® 500-Konjugats

Ein Rutin/Monospher® 500-Konjugat wurde in der folgenden Weise hergestellt.
Die zur Durchführung der Umsetzung verwendete Apparatur wurde mit Stickstoff gespült, um die Luft in der Apparatur zu verdrängen. Dann wurde mit einer Aluminiumverbindung (Di-iso-Butyl-Al) belegtes Monospher® 500 in die Apparatur eingebracht und mit THF angeschlämmt, um eine 10%ige Dispersion herzustellen. Danach wurde die

folgende Verbindung in die Dispersion eingebracht:

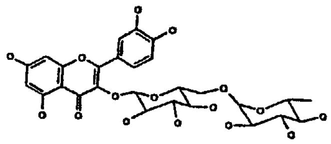

Die erhaltene gelbe Reaktionslösung wurde über Nacht bei Raumtemperatur unter Stickstoffatmosphäre gerührt.

Am nächsten Tag wurde die gelbe Reaktionslösung über ein Druckfilter abfiltriert. Der Filterrückstand wurde dreimal mit je 20 ml THF und/oder Pyridin. Der Nutschkuchen, ein gelbes feines Pulver, wurde über Nacht im Vakuumtrockenschrank bei Raumtemperatur und 150 mbar getrocknet.

Parallel zu dem Versuch wurde noch ein Ansatz über Nacht gerührt, wobei das mit einer Aluminiumverbindung (Di-iso-Butyl-Al) belegte Monospher® 500 durch nicht belegtes Monospher® 500 ersetzt wurde. Das Rutin wurde beim Nachwaschen vollständig vom Monospher® 500 gewaschen, so dass ein weißes Pulver zurückblieb.

In den folgenden Beispielen für Haut- und Sonnenschutzformulierungen wurde das Eusolex® 232/Monospher® 25-Konjugat von Beispiel 1 verwendet. Es ist jedoch auch möglich, ein anderes erfindungsgemäßes Konjugat zu verwenden.

**Beispiel 7 Beispiele für Hautschutzformulierungen**

<u>**Hautschutzlotion (O/W)**</u>

| Rohstoff | INCI | Gew.% | |
|---|---|---|---|
| A Monospher Konjugat | | (1) | 5,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 6,00 |
| Miglyol 812 Neutralöl | Caprylic/Capric Triglyceride | (4) | 6,00 |
| | | | |
| B Propandiol-1,2 | Propylene Gylcol | (1) | 4,00 |
| Allantoin | Allantoin | (1) | 0,20 |
| Konservierungsmittel | | | q.s. |
| Wasser, demineralisiert | Aqua | | ad 100,00 |

<u>Herstellung:</u>

Die Phase A wird auf 75°C und die Phase B auf 80°C erwärmt. Die Phase B wird unter Rühren langsam zu der Phase A gegeben. Das Gemisch wird homogenisiert und unter Rühren abgekühlt.

<u>Bemerkungen:</u>

Viskosität 9200 mPas (Brookfield RVT, Sp. C, 10 Upm) bei 24°C
$pH_{24°C}$=5,0

Konservierungsmittel:

0,05% Propyl-4-hydroxybenzoat
0,15% Methyl-4-hydroxybenzoat

Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg

**Hautschutzlotion (O/W)**

| Rohstoff | INCI | Gew.% | |
|---|---|---|---|
| A Monospher Konjugat | | (1) | 1,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 7,00 |
| Miglyol 812 Neutralöl | Caprylic/Capric Triglyceride | (4) | 7,00 |
| | | | |
| B Propandiol-1,2 | Propylene Gylcol | (1) | 4,00 |
| Allantoin | Allantoin | (1) | 0,20 |
| Konservierungsmittel | | | q.s. |
| Wasser, demineralisiert | Aqua | | ad 100,00 |

Herstellung:

Die Phase A wird auf 75°C und die Phase B auf 80°C erwärmt. Die Phase B wird unter Rühren langsam zu der Phase A gegeben. Das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt

(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg

**Beispiel 8 Beispiele für Sonnenschutzformulierungen**

Sonnenschutzlotion mit IR3535™ (O/W)

| Rohstoff | INCI | Gew.% | |
|---|---|---|---|
| A Monospher Konjugat | | (1) | 3,00 |
| Eusolex 6300 | 4-Methylbenzylidene Camphor | (1) | 3,00 |
| IR 3535™ | Ethyl Butylacetylaminopropionate | (1) | 10,00 |
| (-)-α-Bisabolol | Bisabolol | (1) | 0,30 |
| Montanov 68 | Cetearyl Alcohol (and) Cetearyl Glucoside | (2) | 4,00 |
| Myritol 312 | Caprylic/Capric Triglyceride | (3) | 2,00 |
| Mirasil CM 5 | Cyclomethicone | (4) | 2,00 |
| Mirasil DM 350 | Dimethicone | (4) | 1,00 |
| | | | |
| B Wasser, demineralisiert | Aqua | | ad 100 |
| Glycerin, 87%ig | Glycerin | (1) | 3,00 |
| Konservierungsmittel | | | q.s. |
| | | | |
| C Rhodicare S | Xanthan Gum | (4) | 0,50 |

Herstellung:

Die Phasen A und B werden getrennt voneinander auf 75°C erwärmt. Die Phase C wird bei 75°C unter Rühren langsam zu der Phase B gegeben. Das Gemisch wird gerührt, bis es homogen ist. Anschließend wird die Phase A zu dem Gemisch gegeben. Das Gemisch wird gerührt, bis es homogen ist, und dann unter Rühren abgekühlt.

Bemerkungen:

Konservierungsmittel:

0,05% Propyl-4-hydroxybenzoat
0,15% Methyl-4-hydroxybenzoat
0,30% Germall 115 (ISP, Frechen)

Bezugsquellen:

(1) Merck KgaA, Darmstad
(2) Interorgana, Köln
(3) Henkel, KgaA, Düsseldorf
(4) Rhodia, Frankfurt

**Sonnenschutzmilch (O/W)**

| Rohstoff | INCI | Gew.% | |
|---|---|---|---|
| A Monospher Konjugat | | (1) | 4,00 |
| Eusolex 6300 | 4-Methylbenzylidene Camphor | (1) | 4,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 2,00 |
| Luvitol EHO | Cetearyl Octanoate | (3) | 14,00 |
| Imwitor 900 | Glyceryl Stearate | (4) | 3,00 |
| Cetiol | Oleyl Oleate | (5) | 6,00 |
| Luncacera M | Mikrowax | (6) | 1,00 |
| Miglyol 812 Neutralöl | Caprylic/Capric Triglyceride | (4) | 4,00 |
| | | | |
| B Eusolex 232 | Phenylbenzimidazole Sulfonic Acid | (1) | 2,00 |
| Tris(hydroxymethyl)-aminomethan | Tromethamine | (1) | 1,07 |
| Propandiol-1,2 | Propylene Glycol | (1) | 4,00 |
| Allantoin | Allantoin | (1) | 0,20 |
| Konservierungsmittel | | | q.s. |
| Wasser, demineralisiert | Aqua | | ad 100,00 |
| | | | |
| C Carbopol ETD 2050 | Carbomer ETD 2050 | (7) | 0,25 |
| Wasser, demineralisiert | Aqua | | 30,00 |
| | | | |
| D Tris(hydroxymethyl)-aminomethan | Tromethamine | (1) | 0,25 |
| Wasser, demineralisiert | Aqua | | 4,00 |

Herstellung:

Carbopol ETD 2050 wird in Wasser homogen dispergiert, um die Phase C zu erhalten. Die Phase D wird dann unter Homogenisieren in die Phase C eingetragen. Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Inhaltsstoffe der Phase B zugegeben, und die Phase B wird langsam unter Homogenisieren in die Phasen C/D eingetragen. Die Phase A wird unter Erhitzen gelöst und langsam unter Homogenisieren zugegeben.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg
(7) Goodrich, Neuss

**Sonnenschutzlotion (W/O)**

| | Rohstoff | | Gew.% |
|---|---|---|---|
| A | Monospher Konjugat | (1) | 5,00 |
| | Eusolex HMS | (1) | 5,00 |
| | Eusolex OS | (1) | 5,00 |
| | Eusolex OCR | (1) | 5,00 |
| | Abil WE 09 | (2) | 5,00 |
| | Jojobaöl | (3) | 3,00 |
| | Cetiol V | (4) | 3,00 |
| | Prisorine 2021 | (5) | 2,00 |
| | Lunacera M | (6) | 1,80 |
| | Miglyol 812 Neutralöl | (7) | 3,00 |
| | | | |
| B | Glycerin (etwa 87%) | (1) | 2,00 |
| | Natriumchlorid | (1) | 0,40 |
| | Konservierungsmittel | (1) | q.s. |
| | Wasser, demineralisiert | | ad 100,00 |

Herstellung:

Die Phase B wird auf 80°C und die Phase A auf 75°C erwärmt. Die Phase B wird langsam in die Phase A eingerührt. Das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Th. Goldschmidt AG, Essen

(3) H. Lamotte, Bremen
(4) Henkel KGaA, Düsseldorf
(5) Unichema, Emmerich
(6) H.B. Fuller, Lüneburg
(7) Hüls Troisdorf AG, Witten

**Sonnenschutzcreme (W/O)**

| Rohstoff | INCI | Gew.% | |
|---|---|---|---|
| A Monospher Konjugat | | (1) | 2,50 |
| Eusolex T-2000 | mikron. Titandioxid | (1) | 2,50 |
| Eusolex 6300 | 4-Methylbenzylidene Camphor | (1) | 2,00 |
| Dehymuls E | Dicocoyl Pentyerythrityl Citrate (and) Sorbitan Sesquioleate (and) Cera Alba (and) Aluminium Stearate | (2) | 6.00 |
| Arlacel 989 | PEG-7 Hydrogenated Castor Oil | (3) | 1,00 |
| Bienenwachs | Cera Alba | (1) | 2,00 |
| Zinkstearat | Zinc Stearate | (1) | 2,00 |
| Cetiol J 600 | Oleyl Erucate | (2) | 6,00 |
| Cetiol V | Decyl Oleate | (2) | 6,00 |
| Cetiol OE | Dicaprylyl Ether | (2) | 5,00 |
| Dow Corning 200 (100 cs) | Dimethicone | (4) | 1,00 |
| DL-α-Tocopherolacetat | Tocopheryl Acetate | (1) | 1,00 |
| Vitamin-A-palmitat | Retinyl Palmitate | (5) | 0,50 |
| | | | |
| B Eusolex 232 | Phenylbenzimidazole Sulfonic Acid | (1) | 2,00 |
| Tris(hydroxymethy)-aminomethan | Tromethamine | (1) | 0,88 |
| Glycerin (etwa 87%) | Glycerin | (1) | 5,00 |
| Magnesiumsulfat Heptahydrat | Magnesium Sulfate | (1) | 1,00 |
| Allantoin | Allantoin | (1) | 0,20 |
| Konservierungsmittel | | | q.s. |
| Wasser, demineralisiert | | | ad 100,00 |

Herstellung:

Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Rohstoffe der Phase B zugegeben und auf 80°C erwärmt. Die Phase A wird auf 75°C erwärmt. Die Phase B wird langsam in Phase A eingerührt, und das Gemisch wird unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Henkel KGaA, Düsseldorf
(3) ICI, Essen
(4) Dow Corning, Düsseldorf
(5) Hoffmann La Roche, Schweiz

**Sonnenschutzgel (O/W)**

**mit UV-A/B-Schutz**

| | Rohstoff | | Gew.% |
|---|---|---|---|
| A | Monospher Konjugat | (1) | 2,00 |
| | Eusolex 2292 | (1) | 5,50 |
| | Oxynex K flüssig | (1) | 1,00 |
| | Luvitol EHO | (2) | 9,00 |
| | Dow Corning 200 (100 cs) | (3) | 2,00 |
| | Antaron V-220 | (4) | 2,00 |
| | Jojobaöl | (5) | 5,00 |
| | | | |
| B | Tris(hydroxymethyl)-aminomethan | (1) | 0,60 |
| | Konservierungsmittel | | q.s. |
| | Wasser, demineralisiert | | ad 100,00 |
| | | | |
| C | Pemulen TR-1 | (6) | 0,50 |
| | Wasser, demineralisiert | | 29,50 |
| | | | |
| D | Aloe Vera Gel 1:10 | (7) | 1,00 |

Herstellung:

Das PemulenTR-1 wird im Wasser homogen dispergiert und vorgequollen, um die Phase C zu erhalten. Die Phase B wird unter Homogenisieren in die Phase C eingetragen. Die Phase A wird unter Erwärmen gelöst und langsam unter Homogenisieren zugegeben. Bei 35°C wird die Phase D zugesetzt und nochmals homogenisiert.

Bemerkungen:

Konservierungsmittel:

0,05% Propyl-4-hydroxybenzoat
0,15% Methyl-4-hydroxybenzoat

Bezugsquellen:

(1) E. Merck, Darmstadt
(2) BASF, Ludwigshafen
(3) Dow Corning, Düsseldorf
(4) GAF, Frechen
(5) Henry Lamotte, Bremen
(6) Goodrich, Neuss
(7) Galke, Gittelde

**Sonnenschutzspray (O/W)**

| Rohstoff | INCI | Gew.% | |
|---|---|---|---|
| A Monospher Konjugat | | (1) | 3,00 |
| Eusolex 2292 | Octyl Methoxycinnamate | (1) | 7,50 |
| Eusolex HMS | Homosalate | (1) | 7,00 |
| Volpo S-2 | Steareth-2 | (2) | 0,40 |
| Volpo S-10 | Steareth-10 | (2) | 0,80 |
| Pemulen TR-2 | Acrylate/C 10-30 Alkyl Acrylate Crosspolymer | (3) | 0,18 |
| Hetester PHA | Propylene Glycol Isoceteth-3 Acetate | (4) | 5,00 |
| Performa V 825 | Synthetic Wax | (5) | 0,80 |
| Dow Corning 200 (100cs) | Dimethicone | (6) | 1,00 |
| Oxynex K flüssig | PEG-8 (and) Tocopherol (and) Ascorbyl Palmitate (and) Ascorbic Acid (and) Citric Acid | (1) | 0,10 |
| | | | |
| B Eusolex 232 | Phenylbenzimidazole Sulfonic Acid | (1) | 1,00 |
| Triethanolamin | Triethanolamine | (1) | 0,90 |
| Propandiol-1,2 | Propylene Glycol | (1) | 2,00 |
| Konservierungsmittel | | | q.s. |
| Wasser, demineralisiert | Aqua | | ad 100,00 |

Herstellung:

Zur Neutralisierung von Eusolex 232 wird das Triethanolamin ins Wasser der Phase B gegeben, und Eusolex 232 wird unter Rühren zugegeben. Nach vollständiger Lösung werden die restlichen Rohstoffe der Phase B zugegeben und auf 80°C erwärmt. Die Phase A wird bis auf das Pemulen zusammengegeben und auf 80°C erwärmt. Dann wird das Pemulen in Phase A eingerührt. Die Phase B wird unter Rühren langsam zu der Phase A gegeben, und das Gemisch wird homogenisiert und unter Rühren abgekühlt.

Bemerkungen:

Konservierungsmittel:

0,05% Propyl-4-hydroxybenzoat
0,15% Methyl-4-hydroxybenzoat

Bezugsquellen:

(1) Merck KGaA, Darmstadt
(2) Croda, Nettetal
(3) Goodrich, Neuss
(4) ROVI, Schlüchtem
(5) New Phase, NJ 08554
(6) Dow Corning, Wiesbaden

**Patentansprüche**

1. Konjugat, umfassend ein anorganisches Pigment und einen Wirkstoff auf der Basis organischer Verbindungen, der kovalent über eine Spacer-Gruppe an das anorganische Pigment gebunden ist, **dadurch gekennzeichnet, dass** die Spacer-Gruppe ein Element aus den Gruppen 3A, 4A, 3B, 4B, 5B oder 6B des Periodensystems der Elemente enthält.

2. Konjugat nach Anspruch 1, **dadurch gekennzeichnet, dass** das anorganische Pigment eine Verbindung eines Metalls oder Halbmetalls ist.

3. Konjugat nach Anspruch 2, **dadurch gekennzeichnet, dass** die Verbindung eines Metalls oder Halbmetalls ein Oxid, Silikat, Phosphat, Carbonat, Sulfat oder Nitrid ist.

4. Konjugat nach Anspruch 3, **dadurch gekennzeichnet, dass** das Oxid Magnesiumoxid, Aluminiumoxid, Siliciumoxid, Zinkoxid, Ceroxid, Titanoxid, Zirconiumoxid, Manganoxid, Boroxid, Eisenoxid oder ein Gemisch dieser Oxide ist.

5. Konjugat nach Anspruch 3 oder 4, **dadurch gekennzeichnet, dass** das Oxid in Form von kugelförmigen monodispersen Oxidpartikeln vorliegt.

6. Konjugat nach Anspruch 3, **dadurch gekennzeichnet, dass** das Silikat ein Glimmer oder ein Talk ist.

7. Konjugat nach einem der Ansprüche 1 bis 6, **gekennzeichnet durch** eine Partikelgröße von 1 nm bis 250 μm.

8. Konjugat nach einem der Ansprüche 1 bis 7, **dadurch gekennzeichnet, dass** mindestens zwei Wirkstoffmoleküle an eine Spacer-Gruppe gebunden sind.

9. Konjugat nach einem der Ansprüche 1 bis 8, **dadurch gekennzeichnet, dass** der Wirkstoff eine Verbindung ist, ausgewählt aus lichtabsorbierenden organischen Verbindungen, Substanzen mit antioxidativen und/oder radikalinhibierenden Eigenschaften, Repellentien, Konservierungsmitteln und Derivaten dieser Wirkstoffe.

10. Konjugat nach Anspruch 9, **dadurch gekennzeichnet, dass** die lichtabsorbierende organische Verbindung aus Derivaten von Aminobenzoesäure, Zimtsäure, Salicylsäure, Benzylidenkampfer, Phenylbenzimidazol, Diphenylacrylat, Triazin, Triazol, Benzophenon, Benzoylmethan, Diarylbutadienen und vinylgruppenhaltigen Amiden ausgewählt ist.

11. Konjugat nach Anspruch 10, **dadurch gekennzeichnet, dass** die lichtabsorbierende organische Verbindung photostabil ist.

12. Konjugat nach Anspruch 9, **dadurch gekennzeichnet, dass** die Substanzen mit antioxidativen und/oder radikalinhibierenden Eigenschaften aus Flavonoiden, Coumaranonen, Vitaminen und BHT ausgewählt sind.

13. Konjugat nach Anspruch 9, **dadurch gekennzeichnet, dass** die Repellentien aus Amiden und Derivaten davon ausgewählt sind.

14. Konjugat nach Anspruch 9, **dadurch gekennzeichnet, dass** die Konservierungsmittel aus Benzoesäure und deren

Salzen, Methylparaben, Ethylparaben, Propylparaben, Sorbinsäure und deren Salzen und Salicylsäure und deren Salzen ausgewählt sind.

**15.** Konjugat nach einem der Ansprüche 1 bis 14, **dadurch gekennzeichnet, dass** der Wirkstoff wasserlöslich und/oder öllöslich ist.

**16.** Konjugat nach einem der Ansprüche 1 bis 15, **dadurch gekennzeichnet, dass** die Spacer-Gruppe ein reaktives Metallzentrum umfasst.

**17.** Konjugat nach einem der Ansprüche 1 bis 16, **gekennzeichnet durch** die allgemeine Formel (I)

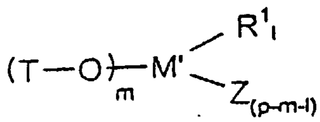

(I)

worin bedeuten:

- T ein Oxid eines Elements, ausgewählt aus der Gruppe Si, B, Al, Fe, Sn, Ti und Zr, oder ein Mischoxid dieser Elemente, im Kern des Pigments befindlich;
- M' ein reaktives Metallzentrum, umfassend ein Element der Gruppen 3A, 4A, 3B, 4B, 5B oder 6B des Periodensystems der Elemente;
- $R^1$ die kovalent gebundene Wirkstoffgruppe;
- Z gleich oder unterschiedlich sein kann und H, OH, ein Halogenatom, ausgewählt aus F, Cl, Br und/oder einen organischen Rest, ausgewählt aus A, OA, ACOO, $NA_2$, SA, gesättigtes oder ungesättigtes Cycloalkyl mit bis zu 6 C-Atomen, heterocyclischer oder aromatischer Rest Ar,

mit

- y 0,1,2,3,4
- A geradkettiger, verzweigter, gesättigter oder ungesättigter Alkylrest mit 1 bis 8 C-Atomen

und

- Ar substituiertes oder unsubstituiertes Phenyl, Naphthyl, Pyridyl, Pyrimidinyl, Thiophenyl, Furanyl, wobei die Substituenten A, OA, COOH, COOA, Cl, F sein können, bedeutet;

- p die maximal mögliche Bindungszahl von M';
- m 1,2 oder 3 und
- l $\leq$ (p-m).

**18.** Konjugat nach Anspruch 17, **dadurch gekennzeichnet, dass** $R^1$ ausgewählt ist aus der Gruppe, bestehend aus

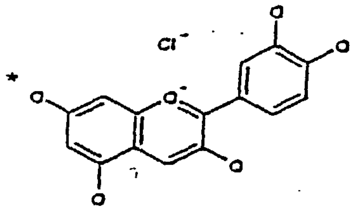

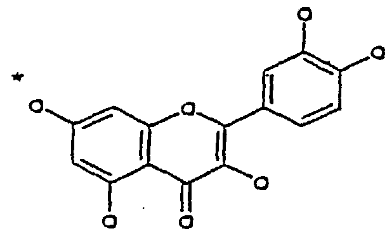

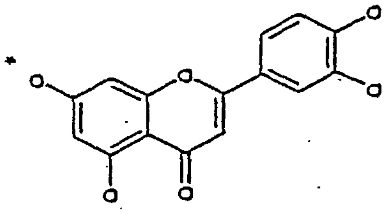

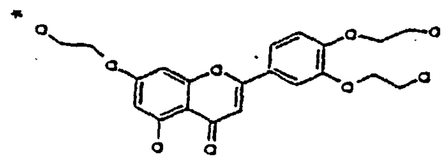

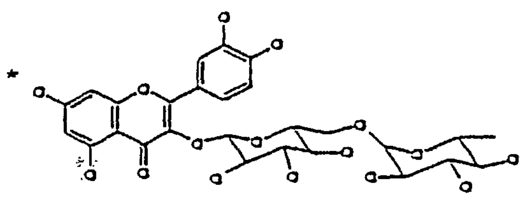

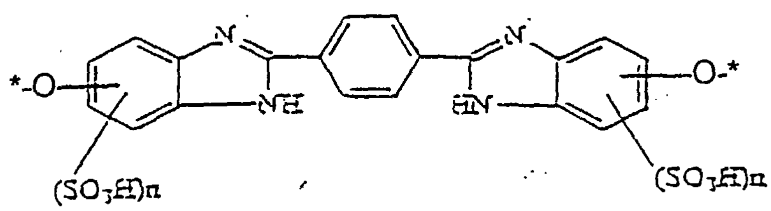

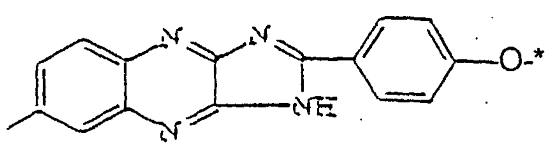

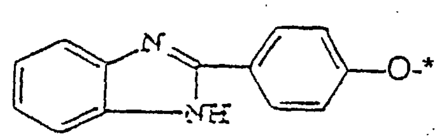

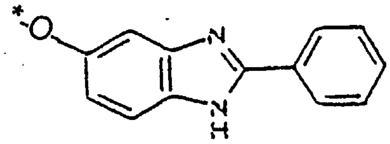

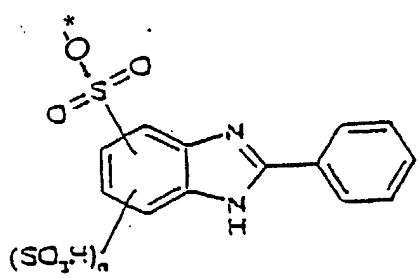

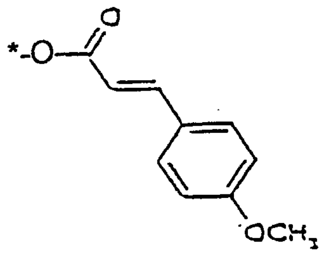

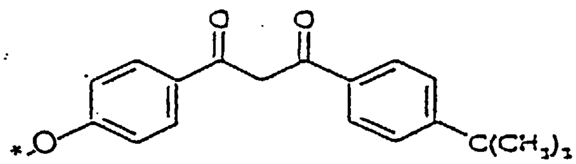

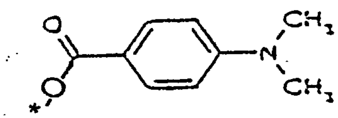

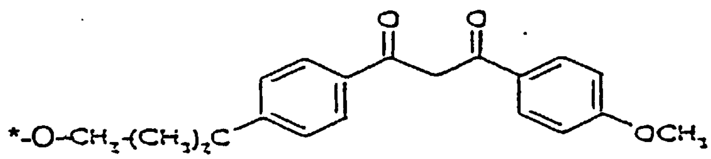

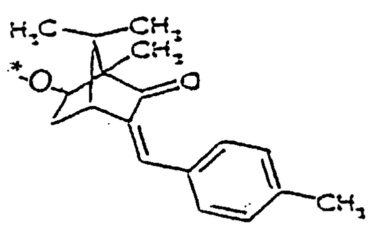

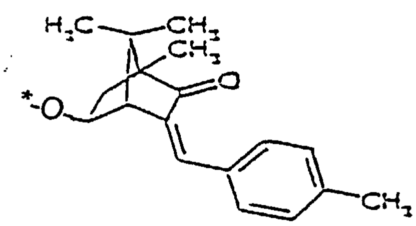

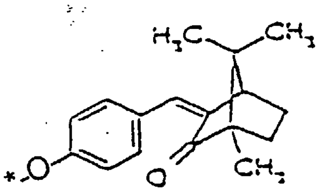

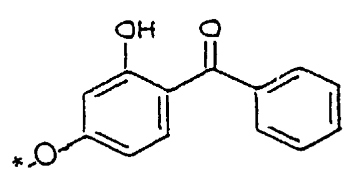

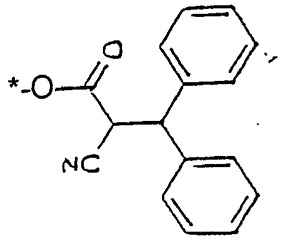

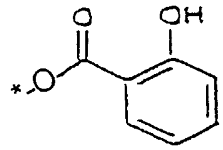

worin n 0,1 oder 2 bedeutet, und * bedeutet die Bindung zur Spacer-Gruppe.

**19.** Konjugat der Formel

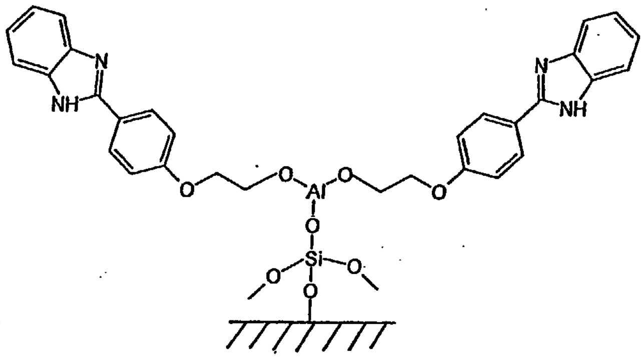

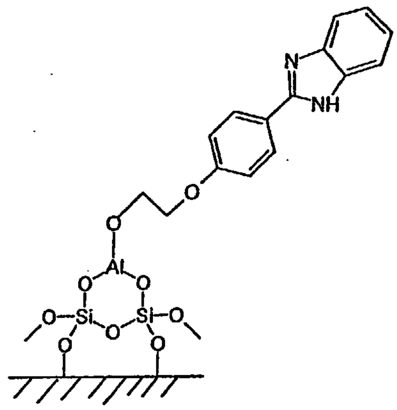

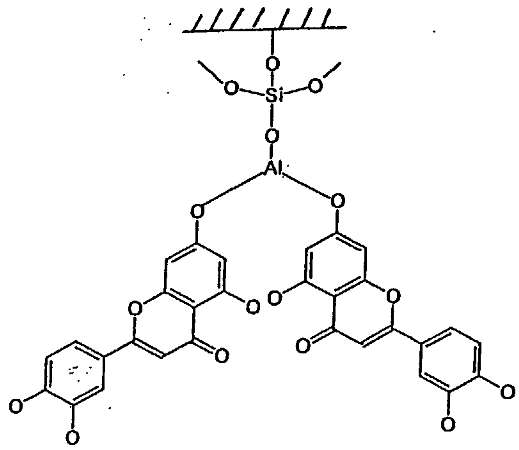

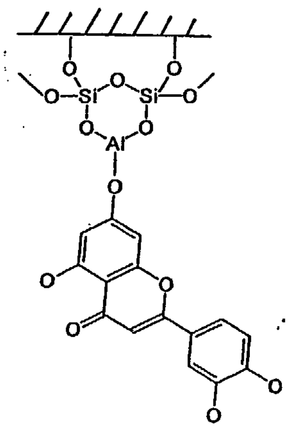

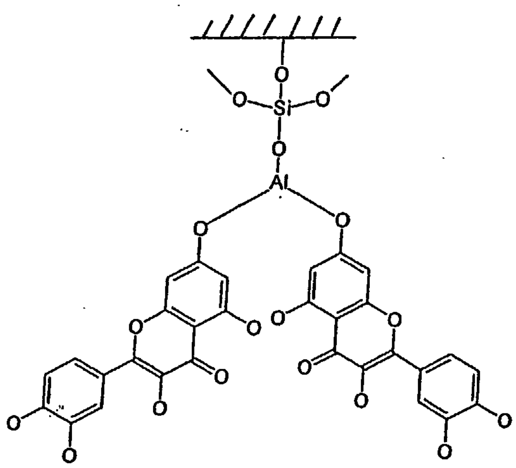

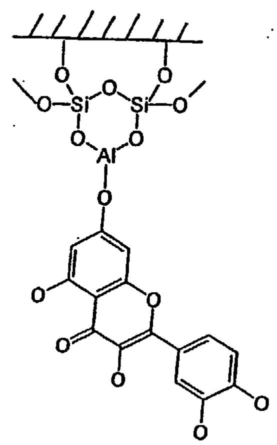

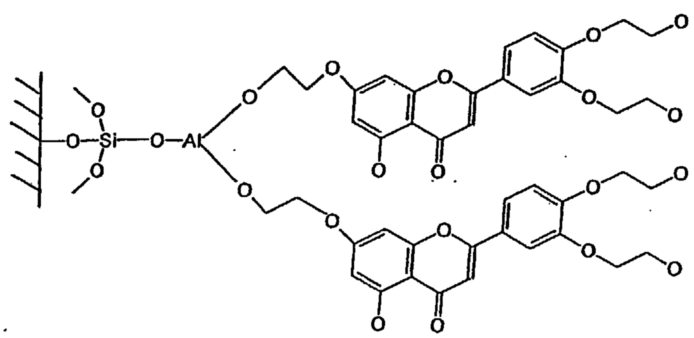

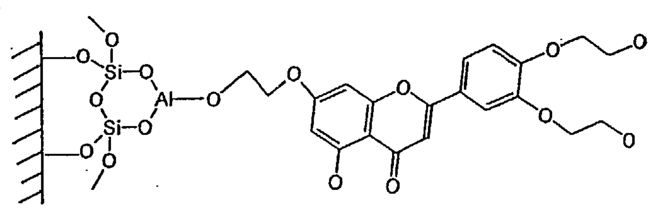

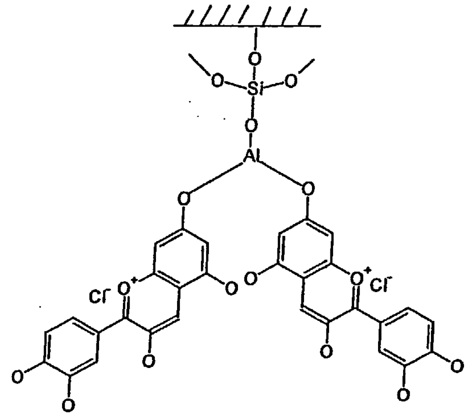

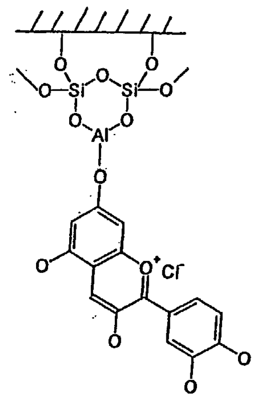

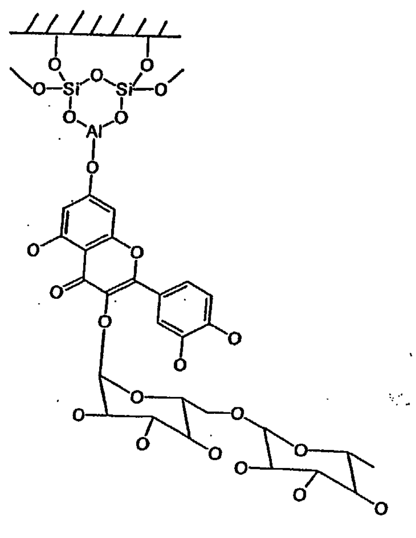

oder

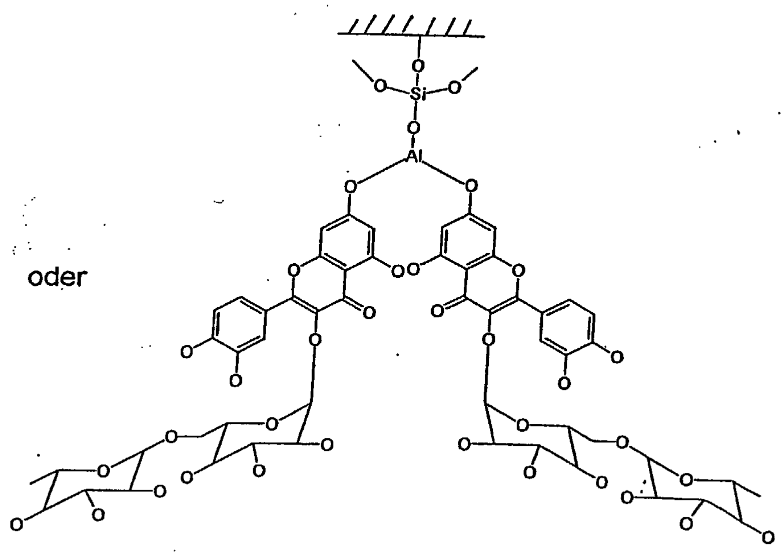

wobei die Strukturen

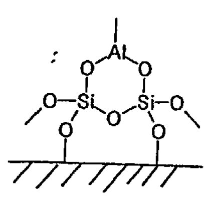

und

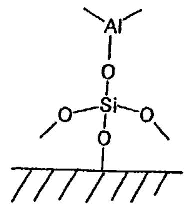

den anorganischen Träger darstellen.

**20.** Konjugat nach einem der Ansprüche 1 bis 16, **dadurch gekennzeichnet, dass** die Spacer-Gruppe eine organische Verbindungsgruppe umfasst.

**21.** Konjugat nach Anspruch 20, **dadurch gekennzeichnet, dass** die organische Verbindungsgruppe durch die Formel $-O(CH_2CH_2O)_n-$ dargestellt ist, worin n eine ganze Zahl im Bereich von 1 bis 20 ist.

**22.** Konjugat nach Anspruch 21, **dadurch gekennzeichnet, dass** n 1 oder 2 ist.

**23.** Dermatologische oder kosmetische Zusammensetzung, umfassend mindestens ein Konjugat nach einem der Ansprüche 1 bis 22 und mindestens einen kosmetisch, pharmazeutisch und/oder dermatologisch verträglichen Träger und/oder Hilfsstoff.

**24.** Dermatologische oder kosmetische Zusammensetzung nach Anspruch 23, **dadurch gekennzeichnet, dass** sie mindestens einen weiteren UV-Schutzstoff enthält.

**25.** Dermatologische oder kosmetische Zusammensetzung nach Anspruch 23 oder 24, **dadurch gekennzeichnet, dass** sie ein Antioxidationsmittel enthält.

**26.** Dispersion, umfassend ein Konjugat nach einem der Ansprüche 1 bis 22 und einen Ölbestandteil und/oder einen flüssigen Lichtfilter.

**27.** Verfahren zur Herstellung des Konjugats nach einem der Ansprüche 1 bis 22, umfassend

(a) das Umsetzen eines Metallderivats, enthaltend ein Element der Gruppen 3A, 4A, 3B, 4B, 5B oder 6B des Periodensystems der Elemente, mit einem anorganischen Pigment, um ein Trägermaterial mit einer reaktiven Oberfläche zu erhalten, und
(b) das Umsetzen des Trägermaterials mit einem kopplungsfähigen Wirkstoff auf der Basis organischer Substanzen, um das Trägermaterial kovalent an den Wirkstoff zu binden.

**28.** Verfahren nach Anspruch 27, **dadurch gekennzeichnet, dass** das Trägermaterial mit einer reaktiven Oberfläche, vor der Durchführung des Schritts (b), weiterhin mit einer kopplungsfähigen organischen Verbindung umgesetzt wird, um das Trägermaterial kovalent über eine organische Verbindungsgruppe an den Wirkstoff zu binden.

**29.** Verwendung des Konjugats nach einem der Ansprüche 1 bis 22 zur Herstellung einer Sonnenschutzformulierung oder einer Hautschutzformulierung.

**30.** Verwendung des Konjugats nach einem der Ansprüche 1 bis 22 zum Schutz der Hautzellen.

**31.** Verwendung nach Anspruch 30 zum Schutz der Langerhans-Zellen in der Haut.

**32.** Verwendung des Konjugats nach einem der Ansprüche 1 bis 22 zum Schutz der DNA.

**33.** Verwendung des Konjugats nach einem der Ansprüche 1 bis 22 zur Immunprotektion.